# EUROPEAN PATENT SPECIFICATION

(11) **EP 3 355 925 B1**
(45) Date of publication and mention of the grant of the patent: **28.02.2024**
(21) Application number: 16782351.7
(22) Date of filing: 30.09.2016
(51) Int. Cl.: A61K 31/4409, A61K 45/06, A61P 9/10, A61P 21/00, A61P 25/00

(54) **TREATMENT OF ACUTE TRAUMATIC INJURY**
BEHANDLUNG VON AKUTEN TRAUMATISCHEN VERLETZUNGEN
TRAITEMENT D'UNE LÉSION TRAUMATIQUE AIGUË

(30) Priority: 01.10.2015 US 201562235874 P
(43) Date of publication of application: 08.08.2018
(73) Proprietor: University of Rochester, Rochester, NY 14642 (US)
(72) Inventor: NOBLE, Mark, Rochester, NY 14618 (US); PRÖSCHEL, Christoph, Pittsford, NY 14534 (US); NATOLA, Heather, Rochester, NY 14620 (US); YUE, Li, Barrington, RI 02806-2933 (US); ELFAR, John C., Rochester, NY 14618 (US); TSENG, Kuang-Ching, Shrewsbury, MA 01545 (US)
(74) Representative: Dehns
(86) International application number: PCT/US2016/054903
(87) International publication number: WO 2017/059309

(56) References cited:
- MCBRIDE J M ET AL: "Dose responses of three 4-aminopyridine derivatives on axonal conduction in spinal cord trauma", EUROPEAN JOURNAL OF PHARMACEUTICAL SCIENCES, ELSEVIER, AMSTERDAM, NL, vol. 27, no. 2-3, 1 February 2006 (2006-02-01), pages 237-242, XP027996700, ISSN: 0928-0987 [retrieved on 2006-02-01]
- RIYI SHI ET AL: "Conduction Block in Acute and Chronic Spinal Cord Injury: Different Dose-Response Characteristics for Reversal by 4-Aminopyridine", EXPERIMENTAL NEUROLOGY, vol. 148, no. 2, 18 June 1997 (1997-06-18) , pages 495-501, XP055333894, AMSTERDAM, NL ISSN: 0014-4886, DOI: 10.1006/exnr.1997.6706
- Anonymous: "Hereditary spinal muscular atrophy - Dog", , 26 March 2013 (2013-03-26), XP055334404, Retrieved from the Internet: URL:http://www.vetbook.org/wiki/dog/index. php?title=Hereditary_spinal_muscular_atrop hy [retrieved on 2017-01-11]
- DARLINGTON C: "Fampridine Acorda Therapeutics.", CURRENT OPINION IN INVESTIGATIONAL DRUGS (LONDON, ENGLAND : 2000) NOV 2000, vol. 1, no. 3, November 2000 (2000-11), pages 375-379, XP008182816, ISSN: 1472-4472

## Description

### FIELD

This disclosure relates generally to compositions for use in treating acute traumatic injury in a subject, in particular, acute traumatic injury in an excitable tissue.

### BACKGROUND

Traumatic injury is a devastating clinical condition for which there is currently no effective treatment. Traumatic injuries to a wide range of tissues can result in lifelong disability for a patient and its effect is enormous in terms of the psychological, social, and financial costs to the patient, the family and society. In the case of traumatic injury to the central nervous system (CNS), the neurological deficits resulting from traumatic injury are often progressive. For instance, CNS injury is frequently followed by brain and/or spinal cord edema that enhances the cascade of injury and leads to further secondary cell death and increased patient mortality. As is the case for injury in all tissues, injury to the CNS is also associated with extensive scarring, and such scarring can impede repair. Thus, methods are needed for the *in vivo* treatment of acute traumatic injury that are successful at providing subsequent trophic support to the remaining tissue, and thus enhance functional repair and recovery, under the complex physiological cascade of events which follow the initial insult. Methods are needed for the *in vivo* treatment of an acute traumatic injury to the CNS and other tissues. Methods are also needed to decrease post-injury scarring, and to decrease other adverse changes in tissue function after traumatic injury. For example, such changes include increases in oxidized lipids indicating a failure of normal tissues processes for elimination of toxic waste products. Such traumatic injuries include injuries to excitable tissues, such as skeletal muscle, cardiac muscle, central nervous system, the visual system, the auditory system or the enteric nervous system and to other tissues, such as damage to gut, liver, pancreas, bone fracture and multiple other examples of traumatic injury. The compositions and methods disclosed herein address these and other needs.

McBride et. al. (McBride J M et. al. (2006), European Journal of Pharmacuetical Sciences, 27(2-3), 237-242) discloses the positive effects following use of 4-aminopridine (4-AP) and three derivatives of (N-methyl, N-ethyl and N-tert-butyl) in an *in vitro* model of axonal conduction in spinal cord trauma.

Riyi Shi et. al. (Riyi Shi et. al. (1997), Experimental Neurology, 148(2), 495-501) discloses the dose-response effects on conduction of the potassium channel blocker 4-aminopyridine in acute and chronic spinal cord injury. Improved conduction is only observed in chronic injury.

Darlington (Darlington C (2000), Current Opinions in Investigational Drugs, 1(3), 375-379) discloses the use of 4-AP in clinical trials and discusses the side effects of 4-AP's use.

### SUMMARY

The present invention is defined by the claims. Any subject-matter falling outside the scope of the claims is provided for information only. Pharmaceutical compositions as defined below are provided herein for use in treating a subject with an acute traumatic injury in a subject; comprising administering to the subject a therapeutically effective amount of said pharmaceutical composition within 2 weeks after the acute after the acute traumatic injury, further comprising repeatedly administering the pharmaceutical composition for between 1 to 16 weeks after the acute traumatic injury. In some embodiments, the injury can be a multi-site and/or multi-organ traumatic injury.

The pharmaceutical composition comprises 4-aminopyridine, a derivative thereof, or a combination thereof, wherein 4-aminopyridine or a derivative thereof is represented by a structure according to Formula I: 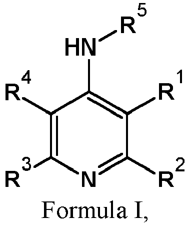 wherein R¹, R², R³, R⁴, and R⁵ are each independently selected from hydrogen, halogen, amine, hydroxyl, alkoxy, carboxyl, or C₁-C₆ alkyl.

In some examples, the pharmaceutical composition can contain 4-aminopyridine and a pharmaceutically acceptable carrier. In some examples, the pharmaceutical composition can contain a derivative of 4-aminopyridine, such as 3,4-diaminopyridine, 3-hydroxy-4-aminopyridine, or a combination thereof, and a pharmaceutically acceptable carrier. In some embodiments, the pharmaceutical composition can be formulated to provide sustained release of the 4-aminopyridine, 4-aminopyridine derivative, or a combination thereof.

The subject in need of treatment can be administered a dose of from about 5 mg/day to about 100 mg/day of 4-aminopyridine, 4-aminopyridine derivative, or a combination thereof. In certain embodiments, the subject can be administered a dose of from about 5 mg/day to about 40 mg/day or about 40 mg/day to about 100 mg/day of 4-aminopyridine, 4-aminopyridine derivative, or a combination thereof.

In some examples, the pharmaceutical composition can contain tetraethyl ammonium and a pharmaceutically acceptable carrier.

The pharmaceutical composition is administered within 2 weeks after the acute traumatic injury. For example, the pharmaceutical composition can be administered within 1 week, within 48 hours, within 24 hours, within 8 hours or within minutes after the traumatic injury. The pharmaceutical composition can be administered repeatedly for about 1 week to about 8 week after the acute traumatic injury. For example, the pharmaceutical composition can be administered repeatedly for about 1 week to about 8 weeks or about 1 week to about 2 weeks after the acute traumatic injury.

The pharmaceutical composition can be administered to the subject by intraperitoneal injection, intravenous injection, intramuscular injection, intrathecal injection, subcutaneous injection, sublingual administration, inhalation, oral administration, transdermal administration, or a combination thereof. In certain embodiments, the pharmaceutical composition can be administered by transdermal administration, implantation, or insertion of a device into the subject. In certain embodiments, the pharmaceutical composition can be administered by injection or orally to the subject.

The pharmaceutical compositions can be administered in combination with an additional therapeutic agent. For example, the pharmaceutical composition can be administered with an anticonvulsant. The anticonvulsant can be selected from lamotrigine, gabapentin, valproic acid, topiramate, famotodine, phenobarbital, diphenylhydantoin, phenytoin, mephenytoin, ethotoin, mephobarbital, primidone, carbamazepine, ethosuximide, methsuximide, phensuximide, trimethadione, benzodiazepine, phenacemide, acetazolamide, progabide, clonazepam, divalproex sodium, magnesium sulfate injection, metharbital, paramethadione, phenytoin sodium, valproate sodium, clobazam, sulthiame, dilantin, diphenylan and L-5-hydroxytrytophan, and a combination thereof.

The compositions for use described herein are used for treating acute traumatic injury in a subject. For example, the compositions for use described herein can be used for treating acute traumatic injury to excitable tissues, such as the CNS in a subject. The central nervous system injury can be a spinal cord injury, a brain injury, or combination thereof. In some embodiments, the injury can be an ischemic injury or reperfusion injury. In some embodiments, the injury can be a crush injury, blast injury, surgical injury, or combination thereof.

In other embodiments, the compositions for use disclosed herein can be used for treating acute traumatic injury to a non-excitable tissue in a subject, such as liver, pancreas bone or other non-excitable tissues. In some embodiments, the injury can be an ischemic injury or reperfusion injury. In some embodiments, the injury can be a crush injury, blast injury, surgical injury, or combination thereof.

In other embodiments, the compositions for use disclosed herein can be used for decreasing the likelihood of adverse consequences following surgical interventions. Such surgical interventions can be to any tissue, including the central nervous system, to cardiac tissue or other excitable tissues, to the gut, to the musculoskeletal system or to other tissues.

In some embodiments, the compositions for use described herein can restore at least a portion of lost motor function or/and sensory function in the subject, enhance repair and regeneration of neural cells such as promote neural cell generation, enhance neural cell survival, reduce glial scarring, or combinations thereof, as compared to an untreated subject. In additional embodiments, the compositions for use described herein can restore at least a portion of function of the injured excitable (but non-neural) tissue (such as skeletal muscle or cardiac muscle), enhance repair and regeneration, enhance cell survival, reduce scarring, decrease other aspects of tissue damage or combinations thereof, as compared to an untreated subject. In further embodiments, the compositions for use described herein can restore at least a portion of function of the injured non-excitable (but non-neural) tissue (such as bone, liver or pancreas), enhance repair and regeneration, enhance cell survival, reduce scarring, or combinations thereof, as compared to an untreated subject.

In some embodiments, the compositions for use disclosed herein can be used for preventing or treating muscle atrophy due to a traumatic injury to the peripheral nervous system, the central nervous system, or the heart.

In some embodiments, the compositions for use disclosed herein can be used for treating acute traumatic injury due to ileus of the gut, e.g. caused by a bowel obstruction, a failure of normal peristalsis, or surgical intervention.

Additional advantages will be set forth in part in the description that follows, and in part will be obvious from the description, or may be learned by practice of the aspects described below. The advantages described below will be realized and attained by means of the elements and combinations particularly pointed out in the appended claims. It is to be understood that both the foregoing general description and the following detailed description are exemplary and explanatory only and are not restrictive.

### BRIEF DESCRIPTION OF THE FIGURES

The accompanying figures, which are incorporated in and constitute a part of this specification, illustrate several aspects described below.

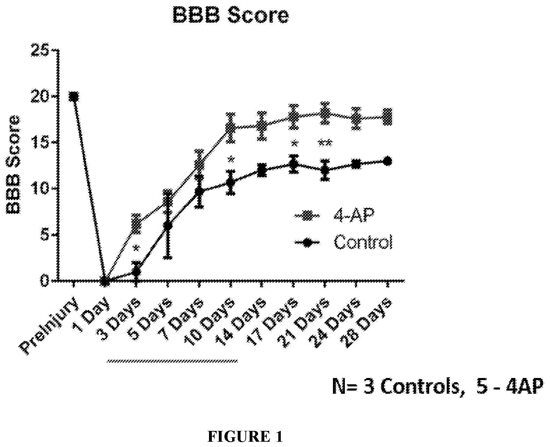

**Figure 1** is a graph demonstrating recovery as measured using the standard Basso, Beattie and Bresnahan (BBB) scores. As shown, there was a significant improvement over placebo (saline) treated rats as early as 3 days after treatment, and a better motor function was observed at every time point examined. When rats were switched to once-a-day administration of 4-AP, and all behavioral analyses were conducted at a time point when the 4-AP half-life in rats predicts that levels in vivo were far below those necessary to observe any effects on motor function, benefits were still readily observable. Moreover, even though treatment was stopped 14 days after injury, the 4-AP treated rats demonstrated durable improvements in motor function.

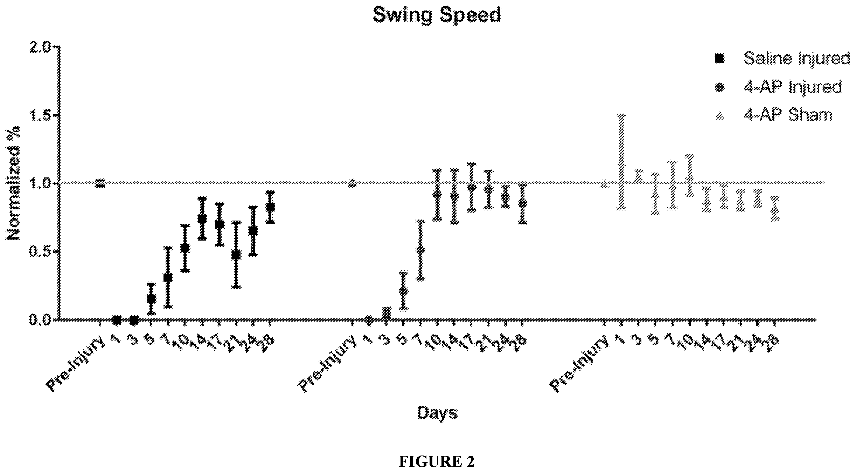

**Figure 2** is a graph that provides information on the speed of the paw during its swing movement (i.e., stride length/swing time). As shown, the placebo (saline)-treated rats do not show a return to normal swing speed (as defined by the group receiving sham surgery with no injury) even over 28 days. In contrast, rats treated with 4-AP showed a return to normal swing speed by 10 days post-injury, and this behavior is maintained when drug treatment ended at day 14.

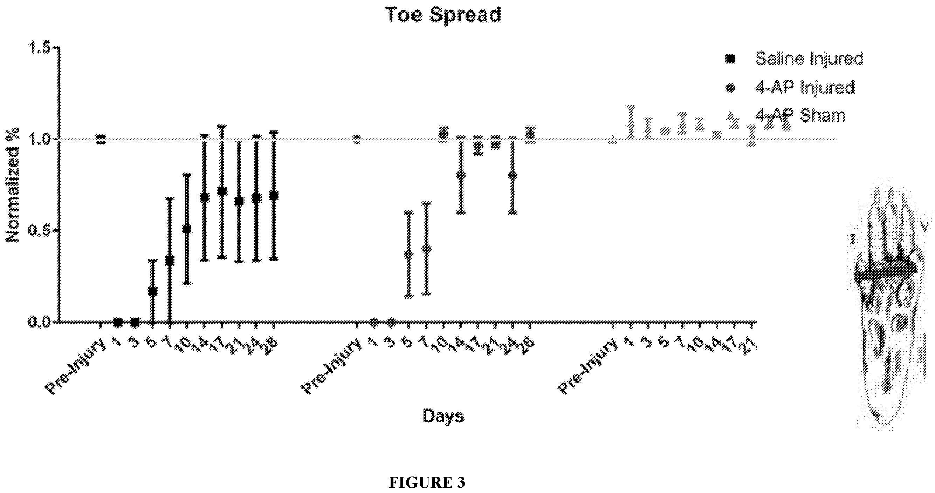

**Figure 3** is a graph that provides information on the distance between the outer toes (i.e., toe spread), a sensitive indicator of normal motor function in the foot itself. Rats treated with placebo (saline) do not return to normal function even over 28 days post-injury. In contrast, rats treated with 4-AP show normal behavior beginning 10 days post-injury, and this behavior is maintained when drug treatment ends at day 14.

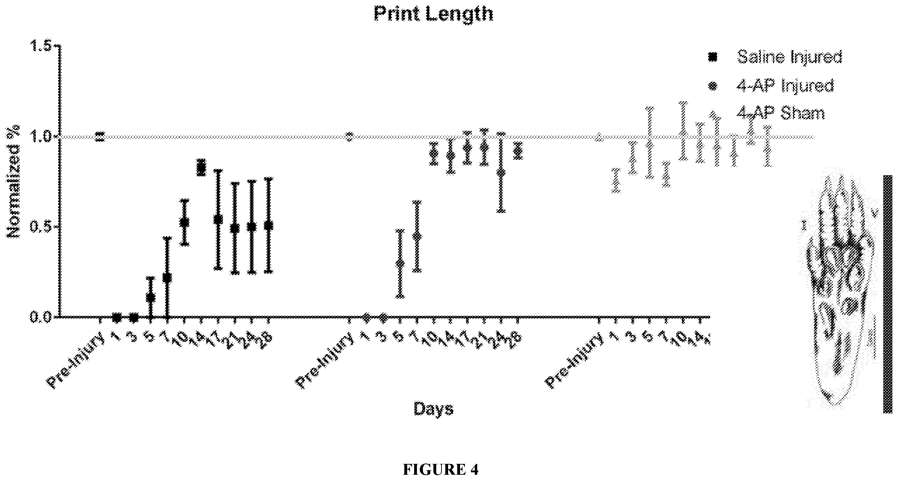

**Figure 4** is a graph that provides information on the length of the foot print, a sensitive indicator of normal motor function in the foot itself. Rats treated with placebo (saline) do not return to normal function even over 28 days post-injury. In contrast, rats treated with 4-AP show normal behavior beginning 10 days post-injury, and this behavior is maintained when drug treatment ends at day 14.

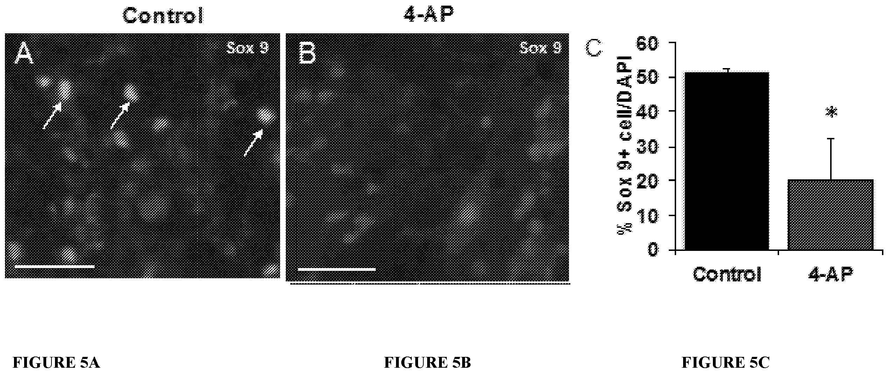

**Figure 5A** and **Figure 5B** are images illustrating a section of control (**Figure 5A**) and 4-AP (**Figure 5B**) treated animals labeled with anti-Sox 9 (bright spots) and DAPI (darker grey). Arrows highlight the cells that were used to define the baseline threshold for Sox 9 immunofluorescence. Size bar represents 20 µm. **Figure 5C** is a graph showing quantification of **Figure 5A** and **Figure 5B** using the threshold shown in A as baseline. N=3, *p=0.045 t-test.

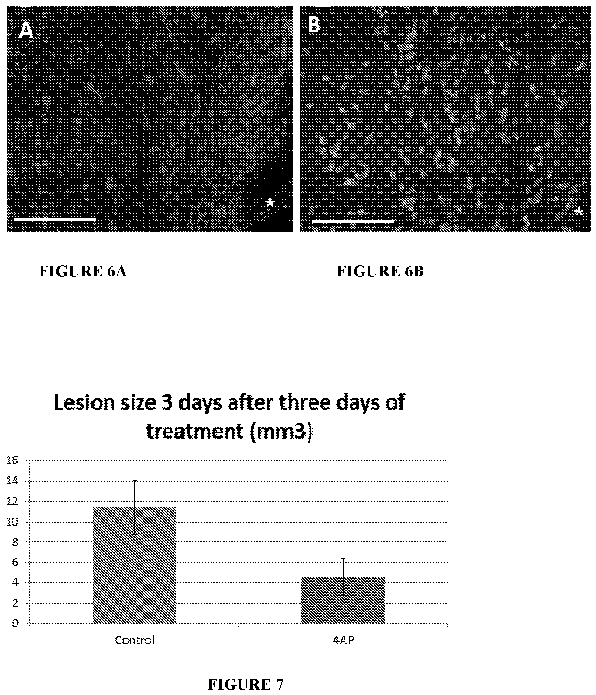

**Figure 6A** and **Figure 6B** are images illustrating a section of spinal cords of placebo (saline) (**Figure 6A**) and 4-AP (**Figure 6B**) treated animals labeled with antibody against glial fibrillary acidic protein (GFAP), which is a marker of scarring in all regions of the central nervous system, (bright spots in **Figure 6A**) and DAPI (bright spots in **Figure 6B**). indicates the lesion edge. The size bar represents 100 µm. As shown in this figure, this canonical marker of central nervous system scarring is greatly decreased in rats that were treated with 4-AP as compared with those treated with saline. These outcomes provide further evidence that 4-AP administration is regulating a response to traumatic injury that is of relevance to traumatic injury in all tissues.
**Figure 7** is a graph showing the unexpected ability of 4-AP to modulate outcomes of traumatic injury that are of relevance to the injury in all tissues of the body is the ability to decrease lesion size. In these experiments, rats received identical injuries as discussed in Example 1, except that these rats were sacrificed 3 days after injury. Lesion size, in cubic millimeters (mm³) was calculated by imaging collected tissue sections covering the full dimensions of the injury using StereoInvestigator and contours were traced to cover the region of the tissue in which damage was apparent. All slides were measured by a blinded individual. Lesion volume was thus determined. As shown in **Figure 7**, 4-AP, applied at clinically relevant concentrations, had the unanticipated property of greatly decreasing lesion size resulting from contusion injury to the spinal cord. Obtaining such a desirable outcome of treatment is relevant to traumatic injury of all types, and there are no known aspects of 4-AP function that would either anticipate such an outcome or would argue that such outcomes were of relevance only to the central nervous system.

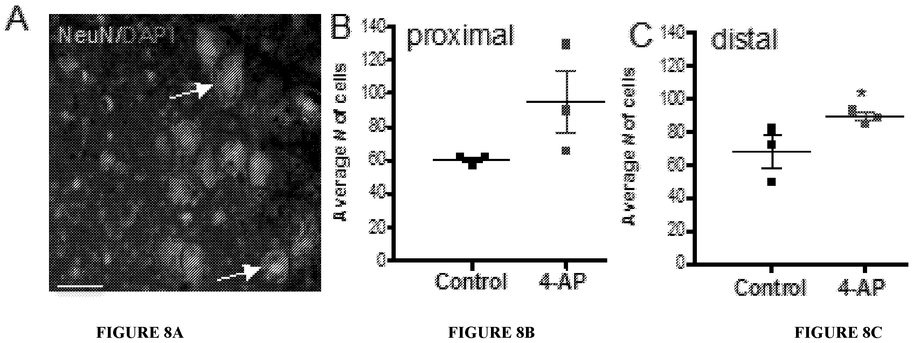

**Figures 8A - 8C** show the rescue of motor neurons by administration of 4-AP in acute traumatic injury to the spinal cord of the central nervous system. Another example of an unanticipated ability of the administration of 4-AP to provide benefit in acute traumatic injury was obtained by examining the number of motor neurons present in spinal cords of rats analyzed 28 days after injury, as in Example 1. Tissue sections were labeled with antibodies to the NeuN antigen, which is a marker of all neuronal populations. Due to the size and location of these neurons, they can only be motor neurons. Large neurons on the ventral side of the cord were then counted, in regions similarly distal or proximal to the lesion site. **Figures 8A-8C** illustrate that 4-AP treated animals have more putative motor neurons. **Figure 8A** is a representative image of NeuN labeled 4-AP treated SCI sections 28 days post injury. The arrows depict large NeuN+ cells defined as putative motor neurons. Size bar represents 20 mm. **Figures 8B** and **8C** are graphs showing quantification of large NeuN+ cells in 1 mm³ region proximal (p=0.06) and distal (p=0.04) to the lesion edge in 4-AP and control sections. N=3.

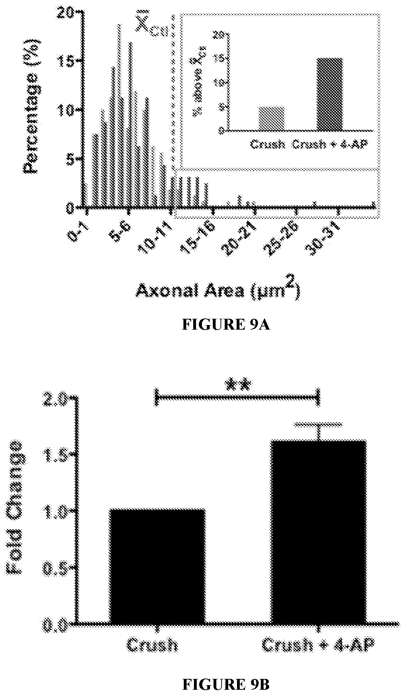

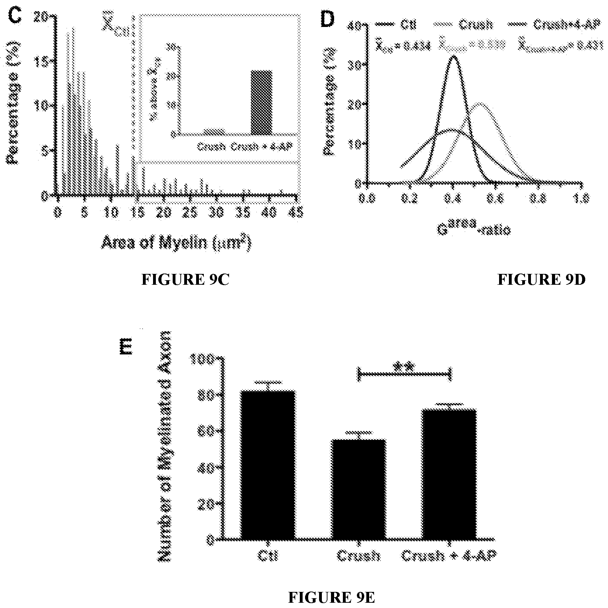

**Figures 9A-9E** show that treatment with 4-AP in acute and subacute periods after traumatic peripheral nerve injury increases axonal area and promotes remyelination. **Figure 9A** shows that sustained local administration of 4-AP increased axonal area following sciatic nerve crush. A comparison of the axonal area of randomly chosen individual axons of vehicle treated and 4-AP treated mice (n=4 for each experimental group; 40 axons analyzed per mouse) is shown. 4-AP treated sciatic nerve showed statistically greater axonal area compared to the vehicle-treated group (p<0.05; ANOVA; restricted-maximum-likelihood). Moreover, 4-AP treated mice had a greater proportion of axons with areas greater than the mean value for uninjured mice, with inset figure displaying all axons with values above this mean. **Figure 9B** shows that 4AP administration in these injuries causes an increase in levels of the P-zero myelin protein, as measured at 21 days post-injury in saline-treated and 4AP treated mice. **Figures 9C and 9D** show that 4-AP administration in these injuries was associated with increased myelin area and close-to-normal G^{area}-ratio compared to untreated group. (**: p<0.01; ANOVA). 4AP treated mice had a greater proportion of axons for which the associated myelin area was greater than the mean value for uninjured mice, with inset figure displaying all axons with values above this mean. **Figure 9E** shows that 4-AP treatment also increased the number of myelinated axons (**:p<0.01; ANOVA).

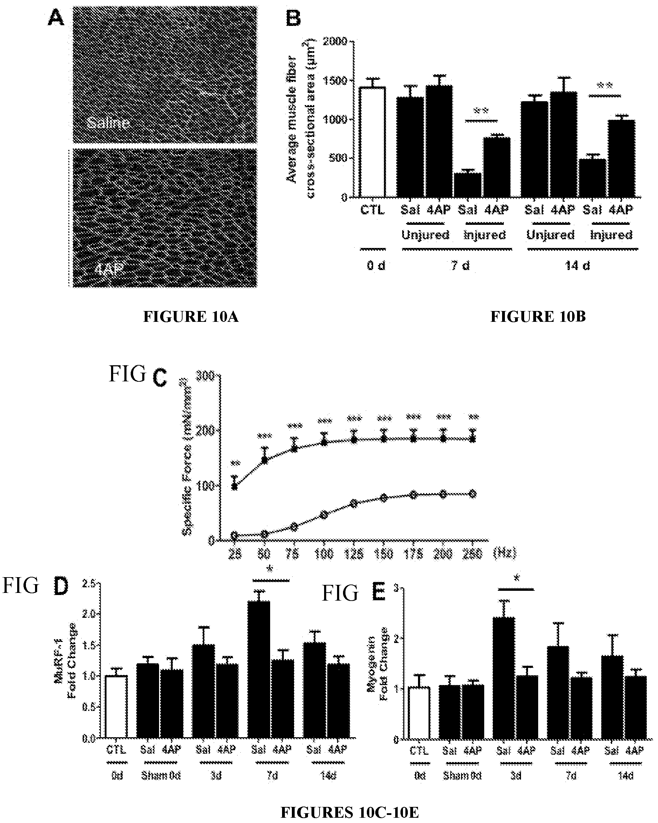

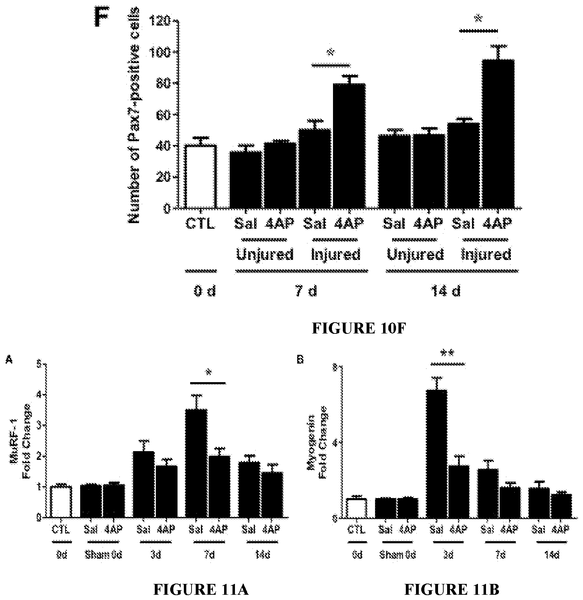

**Figures 10A-10F** show the rescue of skeletal muscle from atrophy induced by crush injury to the sciatic nerve by daily administration of clinically relevant dosages of 4-AP. **Figures 10A****, B** show that 4AP treatment decreases muscle atrophy after sciatic nerve crush injury as judged by decreasing the extent to which cross-sectional size of muscle decreases following crush injury. **Figure 10C** shows that daily treatment with 4-AP also yielded greater muscle strength as compared with saline-treated mice at 7 days after nerve crush injury. * = p<0.05; ** = p<0.01; *** p<0. **Figure 10D** shows that 4-AP treatment decreases expression of MuRF-1, a transcription factor that increases in muscle atrophy. **Figure 10E** shows that 4-AP treatment decreases expression of myogenin, a second muscle transcription factor that also transiently increase during muscle atrophy. **Figure 10F** shows that treatment with 4-AP increased the numbers of Pax7+ cells muscle stem cells both at 7 days and 14 days post nerve crush injury proximal to the muscle. These stem cells are believed to play roles in preventing and/or repairing muscle atrophy.
**Figures 11A-11B** show that daily treatment with 4-AP also decreases muscle atrophy even when the sciatic nerve is cut, thus demonstrating a direct effect of 4AP on skeletal muscle itself that is not nerve dependent. Treatment with 4AP decreased muscle expression of MuRF-1 **(****Figure 11A****)** and myogenin **(****Figure 11B****)** (both normalized to GADPH) after complete denervation. Denervated animals did not show any recovery of gait function, as contrasted with the ability of 4AP to promote functional recovery after nerve crush injury. Thus, the beneficial effects of 4AP on parameters of atrophy were not dependent on restoration of normal motor usage. * = p<0.05; ** p<0.01.

### DETAILED DESCRIPTION

Compositions for use in treating acute traumatic injury in a subject are provided as defined in the claims. In some aspects, the compositions can be used to treat a subject with acute traumatic injury to an excitable tissue, such as the central nervous system.

### General Definitions

Before the present compositions for use are described, it is to be understood that the terminology used herein is for the purpose of describing particular embodiments of the invention.

As used in the specification and the appended claims, the singular forms "a," "an," and "the" include plural referents unless the context clearly dictates otherwise. Thus, for example, reference to "a compound" includes mixtures of various compounds, reference to "the injury" includes one or more injuries, and the like.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed.

"Acute traumatic injury" as used herein refers to the early set of physiological sequelae induced by a traumatic injury. Acute traumatic injury can include both primary (effects arising within minutes to hours) and secondary (effects arising within the next 24 hours to days) physiological effects. Primary and secondary physiological effects are also referred to as acute and sub-acute effects. The compositions and methods described herein can be used to treat both acute and sub-acute traumatic injuries. In certain embodiments, the compositions and methods described herein can be used to treat traumatic injury within a period of time including from the onset of the traumatic injury and up to four to eight weeks after.

"Pharmaceutically acceptable", as used herein, refers to compounds, materials, compositions, and/or dosage forms which are, within the scope of sound medical judgment, suitable for use in contact with the tissues of human beings and animals without excessive toxicity, irritation, allergic response, or other problems or complications commensurate with a reasonable benefit/risk ratio, in accordance with the guidelines of agencies such as the Food and Drug Administration.

A "subject" refers to any individual who is the target of administration or treatment. The subject can be a vertebrate, for example, a mammal. Thus, the subject can be a human or veterinary patient. The term "patient" refers to a subject under the treatment of a clinician, e.g., physician.

"Treating" refers to the administration of a therapeutically effective amount of a therapeutic agent (e.g., 4-aminopyridine) to a subject known or suspected to be afflicted with a traumatic injury or at risk for a traumatic injury due to a surgical procedure. The term "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. It also includes preventative treatment, that is, treatment directed to minimizing or partially or completely inhibiting the development of damage that may be caused by surgical interventions; and supportive treatment, that is, treatment employed to supplement another specific therapy directed toward the durable improvement of the associated disease, pathological condition, or disorder.

A "therapeutically effective amount" refers to that amount of a therapeutic agent that will have a durable beneficial effect, which may be curative, on the health and well-being of the subject with regard to a disease or condition, for example acute traumatic injury, with which the subject is known or suspected to be afflicted. The beneficial effect on the health and well-being of a subject can include, but it not limited to: (1) curing the condition; (2) slowing the progress of the condition; or (3) causing the condition to retrogress. The beneficial effect on the health and well-being of a subject can also include prophylactic outcomes, including but not limited to: (1) preventing or delaying on-set of the condition in the first place; (2) maintaining a condition at a retrogressed level once such level has been achieved by a therapeutically effective amount of a substance; (3) preventing or delaying recurrence of the condition after a course of treatment; or, (4) decreasing the likelihood of tissue damage during surgery.

"Pharmaceutically acceptable excipient" refers to an excipient that is conventionally useful in preparing a pharmaceutical composition that is generally safe, non-toxic, and desirable, and includes excipients that are acceptable for veterinary use as well as for human pharmaceutical use. Such excipients can be solid, liquid, semisolid, or, in the case of an aerosol composition, gaseous.

A "pharmaceutically acceptable carrier" is a carrier, such as a solvent, suspending agent or vehicle, for delivering the disclosed compounds to the patient. The carrier can be liquid or solid and is selected with the planned manner of administration in mind. Liposomes are also a pharmaceutical carrier. As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated.

### Chemical Definitions

Terms used herein will have their customary meaning in the art unless specified otherwise. The organic moieties mentioned when defining variable positions within the general formulae described herein (e.g., the term "halogen") are collective terms for the individual substituents encompassed by the organic moiety. The prefix Cₙ-Cₘ indicates in each case the possible number of carbon atoms in the group.

The term "alkyl," as used herein, refers to saturated straight, branched, primary, secondary or tertiary hydrocarbons, including those having 1 to 20 atoms. In some examples, alkyl groups will include C₁-C₆, C₁-C₅, C₁-C₄, C₁-C₃, or C₁-C₂ alkyl groups. Examples of C₁-C₁₀ alkyl groups include, but are not limited to, methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, heptyl, octyl, 2-ethylhexyl, nonyl and decyl groups, as well as their isomers. Examples of C1-C4-alkyl groups include, for example, methyl, ethyl, propyl, 1-methylethyl, butyl, 1-methylpropyl, 2-methylpropyl, and 1,1-dimethylethyl groups.

The alkyl group can be unsubstituted or substituted with one or more moieties chosen from alkyl, halo, haloalkyl, hydroxyl, carboxyl, acyl, acyloxy, amino, alkyl- or dialkylamino, amido, arylamino, alkoxy, aryloxy, nitro, cyano, azido, thiol, imino, sulfonic acid, sulfate, sulfonyl, sulfanyl, sulfinyl, sulfamonyl, ester, phosphonyl, phosphinyl, phosphoryl, phosphine, thioester, thioether, acid halide, anhydride, oxime, hydrazine, carbamate, phosphoric acid, phosphate, phosphonate, or any other viable functional group that does not inhibit the biological activity of the compounds of the invention, either unprotected, or protected as necessary, as known to those skilled in the art, for example, as described in Greene, et al., Protective Groups in Organic Synthesis, John Wiley and Sons, Third Edition, 1999, hereby incorporated by reference.

The term "alkoxy," as used herein, refers to alkyl-O-, wherein alkyl refers to an alkyl group, as defined above. Similarly, the terms "alkenyloxy," "alkynyloxy," and "cycloalkoxy," refer to the groups alkenyl-O-, alkynyl-O-, and cycloalkyl-O-, respectively, wherein alkenyl, alkynyl, and cycloalkyl are as defined above. Examples of C1-C6-alkoxy groups include, but are not limited to, methoxy, ethoxy, C₂H₅-CH₂O-, (CH₃)₂CHO-, n-butoxy, C₂H₅-CH(CH₃)O-, (CH₃)₂CH-CH₂O-, (CH₃)₃CO-, n-pentoxy, 1 methylbutoxy, 2-methylbutoxy, 3-methylbutoxy, 1,1-dimethylpropoxy, 1,2 dimethylpropoxy, 2,2-dimethylpropoxy, 1-ethylpropoxy, n-hexoxy, 1 methylpentoxy, 2-methylpentoxy, 3-methylpentoxy, 4-methylpentoxy, 1,1 dimethylbutoxy, 1,2-dimethylbutoxy, 1,3-dimethylbutoxy, 2,2-dimethylbutoxy, 2,3 dimethylbutoxy, 3,3-dimethylbutoxy, 1-ethylbutoxy, 2-ethylbutoxy, 1,1,2 trimethylpropoxy, 1,2,2-trimethylpropoxy, 1-ethyl-1-methylpropoxy, and 1-ethyl-2-methylpropoxy.

The term "halogen," as used herein, refers to the atoms fluorine, chlorine, bromine and iodine. The prefix halo- (e.g., as illustrated by the term haloalkyl) refers to all degrees of halogen substitution, from a single substitution to a perhalo substitution (e.g., as illustrated with methyl as chloromethyl (-CH₂Cl), dichloromethyl (-CHCl₂), trichloromethyl (-CCl₃)).

As used herein, the term "substituted" is contemplated to include all permissible substituents of organic compounds. In a broad aspect, the permissible substituents include acyclic and cyclic, branched and unbranched, carbocyclic and heterocyclic, and aromatic and nonaromatic substituents of organic compounds. Illustrative substituents include, for example, those described below. The permissible substituents can be one or more and the same or different for appropriate organic compounds. For purposes of this disclosure, the heteroatoms, such as nitrogen, can have hydrogen substituents and/or any permissible substituents of organic compounds described herein which satisfy the valences of the heteroatoms. This disclosure is not intended to be limited in any manner by the permissible substituents of organic compounds. Also, the terms "substitution" or "substituted with" include the implicit proviso that such substitution is in accordance with permitted valence of the substituted atom and the substituent, and that the substitution results in a stable compound, e.g., a compound that does not spontaneously undergo transformation such as by rearrangement, cyclization, elimination, etc.

Reference will now be made in detail to specific aspects of the disclosed materials, compounds, compositions, articles, and methods, examples of which are illustrated in the accompanying Examples.

### Compositions

The compositions described herein are for use in treating acute traumatic injury in a subject. The compositions comprise a potassium channel blocker as defined below. The term "potassium channel blocker" as used herein refers to a compound which inhibits the potassium channel. Potassium channel blockers are known in the art. Examples of potassium channel blockers which are not part of the present invention include almokalant, ambasilide, amiodarone, azimilide, bepridil, benzocaine, bretylium, bupivacaine, candesartan, canrenoic acid, cetirizine, chloroquine, chlorpheniramine, chromanol 293B, Cibenzoline, cisapride, clofilium, cocaine, diltiazem, diphenhydramine, disopyramide, dofetilide, dronedarone, E-4031, ebastine, eprosartan, flecainide, fluvoxamine, HMR, ibutilide, imipramine, indapamide, irbesartan, ketoconazole, loratadine, losartan, mexiletine, nicotine, nifekalant, nifedipine, papaverine, pentobarbital, pimozide, propafenone, quinidine, risperidone, ropivacaine, RP58866, sematilide, sertindole, d-sotalol, spironolactone, tetraethyl ammonium, tedisamil, terfenadine, terikalant, thioridazine, triamterene, or verapamil.

The composition according to the present invention comprises a potassium channel blocker selected from 4-aminopyridine, a derivative thereof, or a combination thereof, wherein 4-aminopyridine or the 4-aminopyridine derivative has a chemical structure according to Formula I: 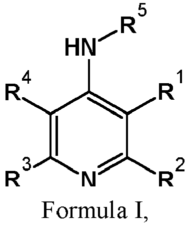 wherein R¹, R², R³, R⁴, and R⁵ can each be independently selected from hydrogen, halogen, amine, hydroxyl, alkoxy, carboxyl, or C₁-C₆ alkyl.

In some examples, R¹, R², R³, R⁴, and R⁵ can all be hydrogen. In some embodiments, the composition can include a pharmaceutically acceptable salt, solvate, or prodrug of 4-aminopyridine or a 4-aminopyridine derivative.

In some examples, the compositions described herein can include 4-aminopyridine. In some examples, the composition can include a derivative of 4-aminopyridine. Representative examples of 4-aminopyridine derivatives include, but are not limited to, 3,4-diaminopyridine, 3-hydroxy-4-aminopyridine, N (4-pyridyl)-t-butyl carbamate, N (4-pyridyl) ethyl carbamate, N (4-pyridyl) methyl carbamate, N (4-pyridyl) isopropyl carbamate, salts thereof, solvates thereof, or prodrugs thereof.

In some aspects, the composition can comprise a tetraethyl ammonium compound or a derivative thereof.

The compositions disclosed herein can be used therapeutically in combination with a pharmaceutically acceptable carrier. The compositions disclosed herein can be conveniently formulated into pharmaceutical compositions composed of one or more of the compositions disclosed herein in association with a pharmaceutically acceptable carrier. See, e.g., Remington's Pharmaceutical Sciences, latest edition, by E.W. Martin Mack Pub. Co., Easton, Pa., which discloses typical carriers and conventional methods of preparing pharmaceutical compositions that can be used in conjunction with the preparation of formulations of the compositions disclosed herein and which is incorporated by reference herein. Such pharmaceutical carriers, most typically, would be standard carriers for administration of compositions to humans and non-humans, including solutions such as sterile water, saline, and buffered solutions at physiological pH. Other compounds will be administered according to standard procedures used by those skilled in the art.

Depending on the intended mode of administration, the pharmaceutical composition can be in the form of, for example, solids, semi-solids, liquids, solutions, suspensions (e.g., incorporated into microparticles, liposomes, etc.), emulsions, gels, or the like, preferably in unit dosage form suitable for single administration of a precise dosage. The pharmaceutical compositions can include, as noted above, an effective amount of the potassium channel blocker 4-aminopyridine compound, a derivative thereof, or a combination thereof. in combination with a pharmaceutically acceptable carrier and, in addition, can include other carriers, adjuvants, diluents, thickeners, buffers, preservatives, surfactants, etc. Pharmaceutical compositions can also include one or more additional active ingredients such as other medicinal agents, pharmaceutical agents, antimicrobial agents, antiinflammatory agents, anesthetics, anti-convulsants, and the like.

Liquid pharmaceutically administrable compositions can, for example, be prepared by dissolving, dispersing, etc., a composition as described herein and optional pharmaceutical adjuvants in an excipient, such as, for example, water, saline aqueous dextrose, glycerol, ethanol, and the like, to thereby form a solution or suspension. If desired, the pharmaceutical composition to be administered can also contain minor amounts of nontoxic auxiliary substances such as wetting or emulsifying agents, pH buffering agents and the like, for example, sodium acetate, sorbitan monolaurate, triethanolamine sodium acetate, triethanolamine oleate, etc. Actual methods of preparing such dosage forms are known, or will be apparent, to those skilled in this art; for example see *Remington's Pharmaceutical Sciences,* referenced above.

Preparations for parenteral administration include sterile aqueous or non-aqueous solutions, suspensions, and emulsions which can also contain buffers, diluents and other suitable additives. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives, such as antimicrobials, anti-oxidants, chelating agents, and inert gases and the like, can also be present.

Parenteral administration, if used, is generally characterized by injection. Injectables can be prepared in conventional forms, either as liquid solutions or suspensions, solid forms suitable for solution or suspension in liquid prior to injection, or as emulsions. A more recently revised approach for parenteral administration involves use of a slow release or sustained release system such that a constant dosage is maintained.

Transdermal formulations can also be prepared in the form of creams, ointments, salves, sprays, gels, lotions, emulsions, and transdermal patches. Such compositions may contain one or more chemical penetration enhancers, membrane permeability agents, membrane transport agents, emollients, surfactants, stabilizers, and combination thereof.

Optionally, the composition may comprise a fibrin glue, a biocompatible polymer or hydrogel or a combination thereof. The compound can be encapsulated in the polymer or hydrogel such that the agent is slowly released in the body to at least one portion of the central nervous system. Optionally, the compound can be dispersed throughout the polymer or hydrogel in such a manner to result in slow, sustained release as the polymer or hydrogel degrades inside the body. In some examples, the composition can comprise a biodegradable biocompatible polymer such as polyglycolide or polyglycolic acid (PGA), polylactide or polylactic acid (PLA), poly-L-lactic acid (PLLA), poly-D/L-lactic acid with polyglycolic acid (PDLLA-co-PGA), poly-L-lactic acid-co-glycolic acid (PLGA), PDLLA with bioactive glass, PLGA with bioactive glass, poly-L-lactic acid with β-tricalcium phosphate (PLLA-TCP), poly-L-lactic acid with hydroxyapatite (PLLAHA), polydioxanone (PDS), polyethylene glycol (PEG), poly(8-caprolactone) (PCL), polycaprolactone (PCL) with alginate, polyhydroxybutyrate (PHB), polycarbonate (PC), N-vinyl pyrrolidone copolymers, polyorthoester, chitosan, poly(2-hydroxyethyl-methacrylate) (PHEMA), hyaluronic acid and hydrogels.

In some examples, the composition can be in the form of beads, films, or some other shape, as would be understood by a person of ordinary skill in the art. The size of each individual bead, film, or other shape is of a suitable size for implantation or other form of administration, as would be understood by a person of ordinary skill in the art. Further, the size of each individual bead, film, or other shape may be substantially consistent, or there may be a distribution of different sizes of the respective shape. Optionally, the beads can be implanted, ingested, or otherwise placed inside the body in some way, such that the agent is administered locally or systemically in a sustained-release manner.

Dry powder formulations ("DPFs") with large particle size have improved flowability characteristics, such as less aggregation, easier aerosolization, and potentially less phagocytosis. Dry powder aerosols for inhalation therapy are generally produced with mean diameters primarily in the range of less than 5 microns, although a preferred range is between one and ten microns in aerodynamic diameter. Large "carrier" particles (containing no drug) have been co-delivered with therapeutic aerosols to aid in achieving efficient aerosolization among other possible benefits.

The pharmaceutical composition can contain from about 0.01 to about 99 percent of 4-amino pyridine or a derivative thereof, together with the carriers and/or excipients. For example, the amount of 4-aminopyridine, a derivative thereof, or a combination thereof, by weight of the pharmaceutical composition can be about 0.1% or greater, about 1% or greater, about 2% or greater, about 5% or greater, about 10% or greater, about 15% or greater, about 20% or greater, about 25% or greater, about 30% or greater, about 50% or greater, about 75% or greater, or about 90% or greater. In some embodiments, the pharmaceutical composition can be a solution comprising from about 10 nM to about 1 µM 4-aminopyridine, a 4-aminopyridine derivative, or a combination thereof.

The pharmaceutical compositions described herein are used in a "therapeutically effective amount" of the 4-aminopyridine, a derivative thereof, or a combination thereof. In some embodiment, the pharmaceutical composition can be formulated, such that when administered, it delivers a therapeutically effective amount of 4-aminopyridine, a derivative thereof, or a combination thereof in an amount of 5 mg or greater. For example, the pharmaceutical composition when administered can deliver 6 mg or greater, 7 mg or greater, 7.5 mg or greater, 8 mg or greater, 9 mg or greater, 10 mg or greater, 15 mg or greater, 20 mg or greater, 25 mg or greater, 30 mg or greater, 35 mg or greater, 40 mg or greater, 45 mg or greater, 50 mg or greater, 55 mg or greater, 60 mg or greater, 65 mg or greater, 70 mg or greater, 75 mg or greater, 80 mg or greater, 85 mg or greater, 90 mg or greater, or 95 mg or greater of 4-aminopyridine, a derivative thereof, or a combination thereof.

### Methods

Methods of treatment of the human body by therapy, referred to in this description, are not part of the present invention as such, but are described here in relation to compounds and pharmaceutical compositions for use in said methods for treatment of the human body by therapy, according to the present invention.

The compounds and compositions described herein are used to treat acute traumatic injury in a subject. In some aspects, the compounds and compositions can be used to decrease tissue damage and/or enhance recovery during the acute and/or subacute post-injury period. In certain embodiments, the compounds and compositions can be used to treat acute traumatic injury in an excitable tissue of the subject. "Excitable tissue" as used herein refers to tissues that contain excitable cells. Excitable cells are cells that respond actively to an electric stimulus and have an electrical charge differential across their cellular membranes. Excitable cells are generally capable of undergoing an action potential. Such cells can express channels, such as voltage-gated, ligand-gated, and stretch channels, which allow flow of ions (potassium, sodium, calcium, chloride, etc.) across the membrane. Excitable tissue can include nervous tissue and muscle tissue, including cardiovascular tissue. In some embodiments, the compounds and compositions described herein can be used to treat acute traumatic injury in an excitable tissue, wherein the excitable tissue is not a tissue of the peripheral nervous system (e.g., is a tissue of the central nervous system, the eye, the ear, or is a type of muscle). In some further examples, the excited tissue is found in the gut.

In some embodiments, the compounds and compositions described herein can be used to treat acute traumatic injury in a non-excitable tissue. For example, the compounds and composition described herein can be used to treat a bone fracture, or tissue during surgery (e.g., hepatectomy).

In some embodiments, the compounds and compositions described herein can be used to decrease Sox9 expression, chondroitin sulfate proteoglycan expression, and other markers of tissue damage after traumatic injury.

In some embodiments, the compounds and compositions described herein can be used to decrease scarring after traumatic injury.

In some embodiments, the compounds and compositions described herein can be used to decrease lesion size after traumatic injury.

In some embodiments, the compounds and compositions described herein can be used to decrease oxidative damage after traumatic injury.

In certain embodiments, the compounds and compositions can be used to treat acute traumatic injury to the central nervous system. For example, the central nervous system injury can be a brain injury and/or spinal cord injury, including complete or partial severance and/or compression and/or reperfusion injury of a region of the spinal cord. The traumatic injury can include injury to the gray matter (including neuronal cell bodies) and white matter (including myelinated axons) of the central nervous system. In some examples, the compositions can be used to treat acute traumatic injury in a subject who is motor complete, sensory complete, motor incomplete, sensory incomplete, or a combination thereof. In other examples, the compositions can be used to treat acute traumatic injury associated with other neurological impairments.

In other embodiments, the compounds and compositions can be used to treat acute traumatic injury to other tissues of the body. For example, the injury can include damage to the eye, the ear, cardiac tissue, to skeletal muscle, to the gut or to smooth muscle in other parts of the body. The injury may also include damage to liver, pancreas, bone or other non-excitable tissues of the body.

In certain embodiments, the compounds and compositions can be used to treat cardiac injuries such as decrease traumatic cardiac damage during the acute and/or subacute post-injury period. Such injuries can be due to myocardial infarction or other types of traumatic damage to the heart, which can cause loss of cardiac tissue, atrophy, and/or fibrotic scarring of cardiac muscle.

In certain embodiments, the compounds and compositions can be used to decrease tissue damage due to a stroke, traumatic brain injury or other head injuries, injuries to the spinal cord (including central cord syndrome), and ischemic injury to muscle, heart or other organs. In these embodiments, the compounds and compositions (such as 4-AP) can treat both the injured tissue and the secondary injuries (such as muscle atrophy) that occur as a consequence of the primary injury.

In certain embodiments, the compounds and compositions can be used to enhance recovery of a bone fracture. The compounds and compositions can be administered during the acute and/or subacute periods.

In certain embodiments, the compounds and compositions can be used to prevent traumatic injury caused by burns. The compounds and compositions can be administered during the acute and/or subacute periods to decrease damage and/or enhance repair of damage.

In certain embodiments, the compounds and compositions can be used to prevent traumatic injury caused to the kidney, such as may occur due to ischemic injury, in automobile accidents, or other situations. The compounds and compositions can be administered during the acute and/or subacute periods.

In certain embodiments, the compounds and compositions can be used to treat multi-site and/or multi-organ traumatic injury. For example, traumatic injury to the spinal cord or the brain is often associated with damage to peripheral nerves and to skeletal muscle and other tissues. The use of the compounds and compositions disclosed herein provides a method to treatment such multi-site and/or multi-organ traumatic injuries due to their ability to promote recovery in multiple tissues.

In certain embodiments, the compounds and compositions can be used to treat acute traumatic injury due to ileus of the gut, e.g. caused by a bowel obstruction, a failure of normal peristalsis, surgical intervention or other reasons. The compounds and compositions can be administered during the acute and/or subacute periods.

The traumatic injury can be mild to severe. In some embodiments, the traumatic injury can be the result from an auto or other accident, a stroke-induced lesion, ischemic injury, reperfusion injury, crush injury, blast injury, injury from a surgical procedure, and combinations thereof. In some embodiments, the acute traumatic injury is not to the peripheral nervous system.

The pharmaceutical composition can be administered by any suitable method depending on whether local or systemic treatment is desired, and on the area to be treated. For example, the pharmaceutical composition can be administered to a subject topically, intranasally, orally, or parenterally. In some examples, the pharmaceutical composition can be administered by intradermal, implantation, subcutaneous, transdermal, intramuscular, intraperitoneal, intrarectal, intraarterial, intralymphatic, intravenous, intrathecal, and intratracheal routes.

In certain embodiments, the pharmaceutical composition can be inserted, implanted, or injected into the subject. For example, the pharmaceutical composition can be inserted, implanted, or injected at or near the site of the traumatic injury. Thus, the pharmaceutical compositions can be provided locally at the site of the traumatic injury. Optionally, beads, films, or other form of slow-release mechanism can be placed in relatively close proximity to an injured tissue (e.g., a nerve in the central nervous system), such that the agent is administered in a sustained-release manner to the injured tissue. Optionally, the compositions are placed in relatively close proximity to the injured tissue prior to, or concurrent with, a surgical procedure, for example by way of image guidance. By relatively close proximity includes but is not limited to abutting the site of injury or within 1-2 centimeters of the site of injury.

In certain embodiments, the pharmaceutical composition can be administered by delivery devices that are known to those of skill in the art. For example, the composition can be administered by a sustained delivery device or inert delivery vehicle. The sustained delivery device can be an implant or osmotic pump. Optionally, the implant is a nerve cuff. In some embodiments, the implant can be a bioerodible or reservoir-based implant. Sustained delivery devices in which the compositions and/or agents can be incorporated are known. In some embodiments, the compositions can be used to coat the implantable device. For example, the disclosed compositions could be used to coat the rough surface of an implantable device to enhance the compatibility of the device by providing a biocompatible smooth surface which reduces the occurrence of abrasions from the contact of rough edges with the adjacent tissue.

In some embodiments, the pharmaceutical composition can be administered transmucosally, transepithelially, transendothelially, or transdermally. In some examples, the pharmaceutical composition can be administered transdermally. In some embodiments, the pharmaceutical compositions can be topically applied to the skin, for example in the vicinity of the area to be treated.

In some embodiments, the pharmaceutical composition is administered in periodic doses over a given period. Alternatively, the pharmaceutical composition is administered as a sustained release formulation where the pharmaceutical is continuously released to the subject over a given period (e.g., an implant or transdermal patch).

In some embodiments, administration of the pharmaceutical composition involves delivery of at least 5%, 10%, 15%, 20%, 25%, 30%, 35%, 40%, 45%, 50%, 55%, 60%, 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 20 96%, 97%, 98%, 99% of potassium channel blocker (i.e., 4-aminopyridine, a 4-aminopyridine derivative, or a combination thereof) dosage beginning within 1, 5, 10, 15, 20, 30, 45, or 60 minutes of administration. In other embodiments, the concentrations of the potassium channel blocker may be delivered over the course of 1-24 hours.

The selected dosage level of the po4-aminopyridine, a derivative thereof, or a combination thereof will depend upon a variety of factors including the activity of the 4-aminopyridine or the particular derivative, the route of administration, the time of administration, the rate of excretion or metabolism of the particular compound being employed, the duration of the treatment, other drugs and/or materials used in combination with the particular antioxidant employed, the age, sex, weight, condition, general health and prior medical history of the patient being treated, the possibility of co-usage with other therapeutic treatments including use of other therapeutic agents, and like factors well-known in the medical arts. A broad range of disclosed composition dosages are believed to be both safe and effective. In some examples, effective amounts of 4-aminopyridine, a derivative thereof, or a combination thereof for treating a mammalian subject can include about 5 mg to about 40 mg per day (e.g., from about 10 mg to about 40 mg per day; from about 5 mg to about 30 mg per day; or from about 5 mg to about 20 mg per day). In some examples, the therapeutically effective amount of 4-aminopyridine, a derivative thereof, or a combination thereof is about 10 mg per day. A physician or veterinarian having ordinary skill in the art can readily determine and prescribe the effective amount of the pharmaceutical composition required. For example, the physician or veterinarian could start doses of the composition and increase or decrease the levels as required in order to achieve the desired therapeutic effect. This is considered to be within the skill of the artisan and one can review the existing literature on a specific compound or similar compounds to determine optimal dosing.

The pharmaceutical composition is administered within two weeks or less of the traumatic injury. In some examples, the pharmaceutical composition can be administered within about 10 days or less, 8 days or less, 7 days or less, 6 days or less, 5 days or less, 4 days or less, 3 days or less, 48 hours or less, about 42 hours or less, about 36 hours or less, about 24 hours or less, about 18 hours or less, about 12 hours or less, about 6 hours or less, about 5 hours or less, about 4 hours or less, about 3 hours or less, about 2 hours or less, or about 1 hour or less of the traumatic injury. In some examples, the pharmaceutical composition can be administered within about 1 hour to 2 weeks, about 1 hour to 1 week, about 1 hour to 5 days, about 1 hour to 4 days, about 1 hour to 3 days, about 1 hour to 2 days, about 1 hour to 36 hours of the traumatic injury. In some examples, the pharmaceutical composition can be administered after about 12 hours, about 18 hours, about 24 hours, about 30 hours, about 36 hours, about 42 hours, or about 48 hours, of the traumatic injury. In some examples, the pharmaceutical composition can be administered immediately after the traumatic injury.

The compositions described herein are suitable for short term and long term use. "Short-term use", as used herein, can generally refer to the administration to a patient of no more than about 150 doses of the compositions disclosed. Accordingly, the term "long-term use", as used herein, can refer to the administration to a patient of more than about 150 doses of the compounds or compositions disclosed. The compositions can be given as a continuous dose or as a bolus dose, to maximize the circulating levels for the greatest length of time after the dose. Continuous infusion can also be used after the bolus dose. In some examples, the composition can be administered in separate administrations of 2, 3, 4, or 6 equal doses. For example, the about 10 mg to about 40 mg per day can be administered in separate administrations of 2, 3, 4, or 6 equal doses. If more doses are given per day, then the total number of doses that may be given is consequentially larger. Moreover, it is understood that some types of tissue damage may require longer periods of treatment and this timing may be adjusted according the needs of the specific situation being treated. In general, however, the scope of the invention concerns treatment that is initiated during the acute or sub-acute periods following injury and, when terminated, provides durable benefit.

The composition is repeatedly administered for between 1 to 16 weeks after the acute traumatic injury. In certain embodiments, the composition can be repeatedly administered for about 1 week or greater, about 2 weeks or greater, about 3 weeks or greater, about 4 weeks or greater, about 5 weeks or greater, about 6 weeks or greater, about 7 weeks or greater, or about 8 weeks or greater, after the traumatic injury to the central nervous system or other tissues. For example, the composition can be repeatedly administered for about 1 week to about 8 weeks or greater, about 1 week to about 6 weeks or greater, or about 1 week to about 4 weeks or greater, after the traumatic injury to the central nervous system or other tissues.

In some examples, the pharmaceutical composition can be administered within about one week or less of the traumatic injury and treatment continue for up to about 12 weeks. In some examples, the pharmaceutical composition can be administered within about one week or less of the traumatic injury and treatment continue for up to about 8 weeks. In some examples, the pharmaceutical composition can be administered within about 48 hours of the traumatic injury and treatment continue for up to about 8 weeks.

The compounds and compositions described can be administered alone or in combination with one or more additional therapeutic agents. For example, the compositions can be administered with an anticonvulsant or a therapeutic agent used to treat acute traumatic injury to the central nervous system. The one or more additional therapeutic agent may or may not produce a therapeutic effect when administered on its own, but results in such an effect (e.g., pain reduction) when administered with any of the compound or composition disclosed.

The one or more additional therapeutic agents and the compounds and compositions described herein can be administered in any order, including simultaneous administration, as well as temporally spaced order of up to several days apart. The administration of the one or more additional agents and the compounds and compositions described herein can be by the same or different routes. In some examples, the one or more additional agents can be combined with the compounds and compositions described herein.

In some examples, the one or more additional therapeutic agents can include an anticonvulsant. Representative anticonvulsant can include, without limitation, lamotrigine, gabapentin, valproic acid, topiramate, famotodine, phenobarbital, diphenylhydantoin, phenytoin, mephenytoin, ethotoin, mephobarbital, primidone, carbamazepine, ethosuximide, methsuximide, phensuximide, trimethadione, benzodiazepine, phenacemide, acetazolamide, progabide, clonazepam, divalproex sodium, magnesium sulfate injection, metharbital, paramethadione, phenytoin sodium, valproate sodium, clobazam, sulthiame, dilantin, diphenylan and L-5-hydroxytrytophan, and a combination thereof.

When the 4-aminopyridine, a derivative thereof, or a combination thereof is administered with an additional therapeutic agent, the dose can be increased or decreased, depending on the therapeutic agent. For example, when 4-aminopyridine, a derivative thereof, or a combination thereof is administered with an anticonvulsant, the dose can be increased. In some embodiments, the dose of 4-aminopyridine, a derivative thereof, or a combination thereof can be from about 10 mg to about 200 mg per day (e.g., from about 30 mg to about 100 mg per day; from about 40 mg to about 200 mg per day; or from about 40 mg to about 150 mg per day), when administered with an anti-convulsant.

Acute trauma can produce lesions, for example in the CNS that result in dramatic impairment of sensory or motor function. In some embodiments, the compositions for use described herein can result in restoration of at least a portion of lost motor function or sensory function, at least partially lost CNS function, and/or repair and regenerate neural cells in or around a site of central nervous system injury, compared to an untreated subjected. In some embodiments, the compositions for use described herein can enhance neural cell generation, enhance neural cell survival, enhance neural signal transmission capacity in or around a site of CNS damage, reduce scarring, promote formation of new synaptic connections among the cells in or around a site of CNS damage, and a combination thereof. The cells repaired and/or regenerated can include the neural progenitor cells known as neuroepithelial stem cells, neuron-restricted progenitor cells, tripotential glial-restricted progenitor cells, as well as other glial progenitors, astrocytes, and oligodendrocytes. In some examples, the compositions can evoke patterned movement in a subject suffering from an injury to the central nervous system, including movements such as walking, breathing, biking, punching, kicking, swimming, fist clinching, toe pointing, knee bending, hip flexing, sitting, standing, and jumping, driving, and combinations thereof.

### EXAMPLES

The following examples are set forth below to illustrate the compositions, methods, and results according to the disclosed subject matter. These examples are not intended to be inclusive of all aspects of the subject matter disclosed herein, but rather to illustrate representative methods, compositions, and results. These examples are not intended to exclude variations of the present invention, which are apparent to one skilled in the art.

Efforts have been made to ensure accuracy with respect to numbers (e.g., amounts, temperature, etc.) but some errors and deviations should be accounted for. Unless indicated otherwise, parts are parts by weight. There are numerous variations and combinations of reaction conditions, e.g., component concentrations, temperatures, pressures, and other reaction ranges and conditions that can be used to optimize the product purity and yield obtained from the described process. Only reasonable and routine experimentation will be required to optimize such process conditions.

### Example 1:

Experiments were initiated with behavioral analysis of rats receiving contusion injuries to the spinal cord, which is one of the most widely studied of all experimental models of traumatic injury. 8-10 week old Long Evans rats (female) underwent laminectomy at thoracic vertebrae 9 and a 200 kilodyne impact was delivered to the spinal cord using an Infinite Horizons impactor. Animals were allowed to recover on a heated pad and then treatment was started 24 hours after surgery in order to determine whether a clinically useful window of opportunity for initiation of treatment exists for use of this intervention. Animals received 1 mg/kg 4-AP twice a day for seven days, then received 1mg/kg 4-AP for one day for the following seven days. After two weeks, the animals were taken off 4-AP entirely and allowed to continue for 2 weeks to test for stability of recovery.

**Figures 1-4** provide evidence of enhanced recovery of a variety of aspects of motor function. **Figure 1** provides a graph demonstrating recovery as measure using the standard Basso, Beattie and Bresnahan (BBB) scores. As shown, there was significant improvement over placebo (saline) treated rats as early as 3 days after treatment, and a better motor function was observed at every time point examined. Of particular interest were observations that when rats were switched to once-a-day administration of 4-AP and all behavioral analyses were conducted at a time point when the 4-AP half-life in rats predicts that levels in vivo were far below those necessary to observe any effects on motor function, recovery of function was still seen at a time when active levels of 4-AP would not have been present. It is also noteworthy that even though treatment was stopped 14 days after injury, the 4-AP treated rats demonstrated durable improvements in motor function beyond this time point and through the rest of the 28 days of analysis. **Figure** 2 provides information on the speed of the paw during its swing movement (i.e., stride length/swing time). As shown, the placebo (saline)- treated rats do not show a return to normal swing speed (as defined by the group receiving sham surgery with no injury) even over 28 days. In contrast, rats treated with 4-AP showed a return to normal swing speed by 10 days post-injury, and this behavior is maintained when drug treatment ends at day 14. **Figure 3** provides information on the distance between the outer toes (i.e., toe spread), a sensitive indicator of normal motor function in the foot itself. Rats treated with placebo (saline) do not return to normal function even over 28 days post-injury. In contrast, rats treated with 4-AP show normal behavior beginning 10 days post-injury, and this behavior is maintained when drug treatment ends at day 14. **Figure 4** provides information on the length of the foot print, a sensitive indicator of normal motor function in the foot itself. Rats treated with placebo (saline) do not return to normal function even over 28 days post-injury. In contrast, rats treated with 4-AP show normal behavior beginning 10 days post-injury, and this behavior is maintained when drug treatment ends at day 14. All improvements were durable when treatment was stopped at 2 weeks post-injury.

### Example 2:

The ability of daily 4-AP administration to provide benefit at the tissue level was investigated, leading to the wholly unexpected discovery of effects of 4-AP that are relevant to traumatic injuries in a wide range of tissues. The first such observation came from examination of the expression of Sox 9, a transcription factor that is expressed in a wide range of tissues of the nervous system, other excitable tissues and tissues that are not considered to be excitable (including, for example, pancreas, lung, chondrocytes, cartilage, liver, breast, prostate, colorectal, intestine and testis). Among other roles, Sox 9 controls production and expression of chondroitin sulfate proteoglycan, a critical component of scar in many different tissues. Tissue scarring is itself a major impediment to full return of function in all tissues, and any intervention that would be able to modulate expression of Sox9, chondroitin sulfate proteoglycan expression, and other markers of tissue damage, and thus decrease scarring reactions, would be of great relevance in all types of traumatic injury.

**Figures 5A-5C** demonstrates that one of the effects of 4-AP administration in rats with contusion injuries to the spinal cord is to greatly decrease the expression of Sox9, an outcome that cannot be predicted either by any previous analyses of the effects of 4-AP or by the striking behavioral recovery discussed in Example 1.

**Figures 6A-6B** further examines expression of a protein called glial fibrillary acidic protein (GFAP), which is one of the markers of scarring in all regions of the central nervous system. As shown in this figure, this canonical marker of central nervous system scarring is greatly decreased in rats that were treated with 4-AP as compared with those treated with saline. These outcomes provide further evidence that 4-AP administration is regulating a response to traumatic injury that is of relevance to traumatic injury in all tissues.

### Example 3:

4-aminopyridine administration decreases auto-fluorescence following contusion injury to the spinal cord. One of the responses to tissue injury in a broad variety of tissues is the accumulation of substances that may be given the general name of lipofuscin, a name given to lipid-containing residues of lysosomal digestion. This pigment is thought to be the product of oxidation of unsaturated fatty acids and is considered indicative of membrane damage of a nature that occurs widely in tissue injury, a variety of diseases and in aging. Lipofuscin is most readily observed as an auto-fluorescent component of tissue that is seen when tissue is excited with a wide range of emission wavelengths normally used for studying fluorescently labeled proteins, such as antibodies, that are widely used in studying all of the tissues of the body. Lipofuscin accumulation has been reported for cell types of a wide range of tissues, particularly after injury, including liver, kidney, heart muscle, retina, adrenal gland, nerve cells, and red-blood cells, in post-traumatic arthritis and in other tissues.

It was observed in these studies that in rats receiving contusion injuries to the spinal cord, an example of a severe traumatic tissue injury, the background fluorescence of the tissue is greatly increased, even to the extent of requiring special fixation techniques in order to be able to label tissues for standard fluorescence-based immunohistological analyses.

In contrast, it was found that rats treated with 4-AP showed little evidence of lipofuscin accumulation. This is another wholly unexpected ability of 4-AP to beneficially modulate an outcome of traumatic injury that is applicable to a wide range of different tissues.

### Example 4:

Still another example of the unexpected ability of 4-AP to modulate outcomes of traumatic injury that are of relevance to such injury in all tissues of the body is the ability to decrease lesion size. In these experiments, rats received identical injuries as discussed in Example 1, except that these rats were sacrificed 3 days after injury. Lesion size, in cubic millimeters (mm³) was calculated by imaging collected tissue sections covering the full dimensions of the injury using StereoInvestigator and contours were traced to cover the region of the tissue in which damage was apparent. All slides were measured by a blinded individual. Lesion volume was thus determined.

As shown in **Figure** 7, 4-AP, applied at clinically relevant concentrations, had the unanticipated property of greatly decreasing lesion size resulting from contusion injury to the spinal cord. Obtaining such a desirable outcome of treatment is relevant to traumatic injury of all types, and there are no known aspects of 4-AP function that would either anticipate such an outcome or would argue that such outcomes were of relevance only to the central nervous system.

### Example 5:

Rescue of motor neurons by administration of 4-AP in acute traumatic injury to the spinal cord of the central nervous system. Another example of an unexpected ability of the administration of 4-AP to provide benefit in acute traumatic injury was obtained by examining the number of motor neurons present in spinal cords of rats analyzed 28 days after injury, as in Example 1. Tissue sections were labeled with antibodies to the NeuN antigen, which is a marker of all neuronal populations. Due to the size and location of these neurons, they can only be motor neurons. Large neurons on the ventral side of the cord were then counted, in regions similarly distal or proximal to the lesion site. In the distal sites, there was a clearly significant increase in the number of motor neurons, as shown in **Figures 8A-8C****,** in regions distal to the injury. There was also a very similar degree of increase in motor neurons in regions proximal to the injury, with power analysis indicating that similar outcomes in a group of 7 animals would be highly statistically significant.

### Example 6

As demonstrated herein, 4-AP treatment following acute injury to peripheral nerves enhances neuronal area, myelination, and a number of myelinated axons. Further, it was demonstrated that treatment with 4-AP following traumatic damage to the peripheral nervous system causes more rapid recovery of motor function and electrophysiological function. Further studies show that 4-AP has the unexpected property of promoting repair of damage to myelin in the peripheral nervous system.

In the experiment below, mice received crush injuries to the sciatic nerve and then a sustained release formulation of 4-AP in a PLGA formulation (4-AP films) was applied locally at the site of injury. At the time that nerve conduction velocity began to recover, nerves were isolated and studied by electron microscopy. It was demonstrated that treatment with 4-AP enhanced both axonal area and myelination. In uninjured myelinated axons, the average area of a myelinated axon (excluding the myelin) was 11.2 +/- 1.3 µm². In injured nerves treated with PLGA film only, the average area of myelinated axons decreased to 5.7 +/- 0.2 µm² (**Figure 9A**). In nerves treated with 4-AP-containing films, the average area of myelinated axons was 6.7 +/- 0.4 µm² (p<0.05 versus mice treated only with film). In addition, the proportion of axons with areas above the average value for uninjured nerves was 5 +/- 3% in injured nerves treated with vehicle alone but increased to 15 +/- 2% in nerves treated with 4-AP containing films (p<0.05).

**Figure 9B** shows that 4-AP administration in these injuries caused an increase in levels of the P-zero myelin protein, as measured at 21 days post-injury in saline-treated and 4-AP treated mice. Analysis of tissue lysates at 21 days post-injury/treatment showed that 4-AP-treated nerves contained 61 +/- 15% more P₀ protein in the lesion area than seen in the nerves in vehicle-treated animals (p<0.05).

Ultrastructural analysis also revealed that localized 4-AP treatment caused significant changes in myelination post-injury. Mice treated with 4-AP-containing films exhibited an increased myelin thickness and area compared with mice treated with PLGA alone. On day 21 after injury, the average thickness of the myelin within the injured area was 0.53 +/- 0.03 µm but was 0.95 +/- 0.14 µm with localized 4-AP administration (p<0.01), as compared with an average myelin thickness in uninjured nerves of 1.26 +/-0.08 µm. In 4-AP treated mice, 21.4 +/- 10.8% of axons had a myelin thicknesses were above the average for uninjured nerve, while vehicle-treated mice had no axons with a myelin thickness above the average. The myelin area was also measured (**Figure 9C****, D**) and it was determined that the average cross-sectional area of myelin per myelinated large axon in uninjured nerve was 14.8 +/- 1.7 µm² 4.9 +/- 0.3 µm² in injured nerve, and was restored to 10.1 +/- 0.7 µm² with localized 4-AP administration (p<0.01). In 4-AP treated mice, 21.2 +/- 9.3% of axons had myelin areas that were above the average for uninjured nerve, while this value in vehicle-treated mice was only 1.9 +/- 1.4%.

The benefits of treatment were also observed by analysis of the ratio of the area of the axon to the area of the axon plus associated myelin. This g^{area} ratio, a variant of the usually employed g-ratio, was calculated due to the decreased circularity in injured nerve. It was shown that at 21 days after injury, the average g^{area}-ratio in crushed nerves increased significantly from 0.43 +/- 0.01 (healthy sciatic nerve) to 0.54 +/- 0.06 (crushed and vehicle-treated sciatic nerve). However, with local 4-AP treatment, the g^{area}-ratio was in the normal range of 0.43 +/- 0.09 (p<0.01) (**Figure 9D**). In addition, in 4-AP treated mice, 54.4 +/- 18.5% of myelinated axons showed a g^{area}-ratio less than the average for uninjured mice, while this value in vehicle-treated mice was 14.4 +/- 1.4%.

The number of myelinated axons was also significantly greater in 4-AP treated mice examined at 21 days post injury, as determined by ultrastructural analysis (Figure 9F). The number of total myelinated axons per TEM grid examined (2310 µm²) was 82 +/- 5 axons in undamaged nerve, as compared with 55 +/- 4 axons in injured vehicle-treated mice and 71+/- 4 axons in 4-AP treated mice (p<0.01 for vehicle-treated vs. 4-AP treated mice).

### Example 7

This example shows that 4-AP can be used to treat the effects of traumatic injury. In particular, 4-AP was applied as a means of preventing skeletal muscle atrophy following damage to the nerve innervating muscles of limb, as illustrated in **Figure 10****.** In these experiments, the sciatic nerve of the mouse was crushed in such a manner that not all axons were severed. In these injuries, skeletal muscle atrophy is a significant consequence, just as it is in patients with similar injuries. This type of injury can be a problem for many reasons, including the fact that if atrophy is not stopped or reversed within a relatively short period of time, the damage done may become irreversible. Administration of 4-AP was shown to be effective in decreasing atrophic changes in skeletal muscle as shown by changes in the cross-sectional area of myotubes **(****Figure 10A**, B), changes in muscle strength **(****Figure 10C****),** and expression of genes and proteins that are markers of skeletal muscle atrophy (**Figure 10** **D,E**). Moreover, administration of 4-AP also caused increases in the number of Pax7-expressing muscle satellite stem cells, which are considered to play an important role in the prevention and or repair of atrophic changes in muscle (**Figure 10F**).

### Example 8

This example shows that 4-AP treatment in acute and subacute setting to prevent atrophic changes in muscle may not only be due to a rescue of nerve function but also mediated by direct effects on skeletal muscle itself. In this example, the nerve innovating the extensor digitorum longus muscle was completely transected 15-25 mm distant from the muscle. 4-AP was then administered during the acute and subacute post-injury periods. As shown in **Figure 11**, even in these situations of complete denervation, administration of 4-AP was able to prevent atrophic changes in skeletal muscle as demonstrated by its effects on the expression of myogenin and MurF1.

### Embodiments:

Embodiment 1. A pharmaceutical composition comprising 4-aminopyridine, or a 4-aminopyridine derivative, or a combination thereof for use in treating an acute traumatic injury in a subject, comprising administering to the subject a therapeutically effective amount of said pharmaceutical composition, repeatedly administering the pharmaceutical composition for between 1 to 16 weeks after the acute traumatic injury and within 2 weeks after the acute traumatic injury;
wherein 4-aminopyridine and the derivative thereof are represented by a structure according to Formula I: 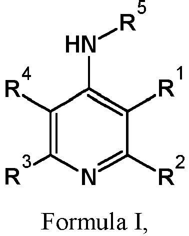 wherein R¹, R², R³, R⁴, and R⁵ are each independently selected from hydrogen, halogen, amine, hydroxyl, alkoxy, carboxyl, or C₁-C₆ alkyl.

Embodiment 2. The composition for use of the preceding embodiment, wherein the acute traumatic injury is to an excitable tissue.

Embodiment 3. The composition for use of any one of the preceding embodiments, wherein the excitable tissue is in the enteric nervous system or in the gut, the auditory system, or the visual system.

Embodiment 4. The composition for use of any one of the preceding embodiments, wherein the excitable tissue is skeletal muscle, cardiac muscle, or smooth muscle.

Embodiment 5. The composition for use of any one of the preceding embodiments, where the acute traumatic injury is to non-excitable tissue.

Embodiment 6. The composition for use of any one of the preceding embodiments, where the non-excitable tissue is bone, liver, pancreas, skin, adrenal gland or lung.

Embodiment 7. The composition for use of any one of the preceding embodiments, wherein the acute traumatic injury is a spinal cord injury, a brain injury, or a combination thereof.

Embodiment 8. The composition for use of any one of the preceding embodiments, wherein the acute traumatic injury is a traumatic cardiac injury.

Embodiment 9. The composition for use of any one of the preceding embodiments, wherein the acute traumatic injury is due to myocardial infarction or other types of traumatic damage to the heart.

Embodiment 10. The composition for use of any one of the preceding embodiments, wherein the acute traumatic injury is due to a stroke, traumatic brain injury or other head injuries, injuries to the spinal cord (including central cord syndrome), and ischemic injury to muscle, heart or other organs.

Embodiment 11. The composition for use of any one of the preceding embodiments, wherein the acute traumatic injury is due to a bone fracture.

Embodiment 12. The composition for use of any one of the preceding embodiments, wherein the acute traumatic injury is due to burns.

Embodiment 13. The composition for use of any one of the preceding embodiments, wherein the acute traumatic injury is to the kidney due to an ischemic injury or an automobile accident.

Embodiment 14. The composition for use of any one of the preceding embodiments, wherein the acute traumatic injury is a multi-site and/or multi-organ traumatic injury.

Embodiment 15. The composition for use of any one of the preceding embodiments, wherein the acute traumatic injury is due to ileus of the gut, as caused by a bowel obstruction, a failure of normal peristalsis, or surgical intervention.

Embodiment 16. The composition for use of any one of the preceding embodiments, wherein the acute traumatic injury is an ischemic injury or reperfusion injury.

Embodiment 17. The composition for use of any one of the preceding embodiments, wherein the acute traumatic injury is a crush injury, blast injury, surgical injury, or combination thereof.

Embodiment 18. The composition for use of any one of the preceding embodiments, wherein the pharmaceutical composition is administered within 2 weeks after the acute traumatic injury.

Embodiment 19. The composition for use of any one of the preceding embodiments, wherein the pharmaceutical composition is administered within 1 week after the acute traumatic injury.

Embodiment 20. The composition for use of any one of the preceding embodiments, wherein the pharmaceutical composition is administered within 24 hours after the acute traumatic injury.

Embodiment 21. The composition for use of any one of the preceding embodiments, further comprising repeatedly administering the pharmaceutical composition for about 1 to about 8 weeks after the acute traumatic injury.

Embodiment 22. The composition for use of any one of the preceding embodiments, wherein the pharmaceutical composition is administered repeatedly for about 1 week to about 4 weeks after the acute traumatic injury.

Embodiment 23. The composition for use of any one of the preceding embodiments, wherein the pharmaceutical composition is administered repeatedly for about 1 week to about 2 weeks after the acute traumatic injury.

Embodiment 24. The composition for use of any one of the preceding embodiments, where repeated administration is by periodic doses of the pharmaceutical composition.

Embodiment 25. The composition for use of any one of the preceding embodiments, where repeated administration is by continuous, sustained release of the pharmaceutical composition.

Embodiment 26. The composition for use of any one of the preceding embodiments, wherein the pharmaceutical composition is administered to the subject by intraperitoneal injection, intravenous injection, intramuscular injection, intrathecal injection, subcutaneous injection, sublingual administration, inhalation, oral administration, or a combination thereof.

Embodiment 27. The composition for use of any one of the preceding embodiments, wherein the pharmaceutical composition is administered by transdermal administration to the subject.

Embodiment 28. The composition for use of any one of the preceding embodiments, wherein the pharmaceutical composition is administered by implantation or insertion of a device into the subject.

Embodiment 29. The composition for use of any one of the preceding embodiments, wherein the pharmaceutical composition provides sustained release of the 4-aminopyridine, 4-aminopyridine derivative, or a combination thereof.

Embodiment 30. The composition for use of any one of the preceding embodiments, wherein the pharmaceutical composition is administered by injection or orally to the subject.

Embodiment 31. The composition for use of any one of the preceding embodiments, wherein the subject is administered the pharmaceutical composition at a dose of from 5 mg/day to 40 mg/day of 4-aminopyridine, 4-aminopyridine derivative, or a combination thereof.

Embodiment 32. The composition for use of any one of the preceding embodiments, wherein the pharmaceutical composition is administered in combination with an additional therapeutic agent.

Embodiment 33. The composition for use of any one of the preceding embodiments, wherein the pharmaceutical composition is administered in combination with an anticonvulsant.

Embodiment 34. The composition for use of any one of the preceding embodiments, wherein the pharmaceutical composition is administered at a dosage level of from about 30 mg to about 200 mg per day in combination with an anticonvulsant.

Embodiment 35. The composition for use of any one of the preceding embodiments, wherein the anticonvulsant is chosen from a barbiturate, a benzodiazepine, a bromide, a carbamate, a carboxamide, a fatty acid, a fructose derivative, a GABA analog, a hydantoin, an oxazolidinedione, a proprionate, a pyrimidinedione, a pyrrolidine, a succinimide, a sulfonamide, a triazine, a urea, or a valproylamide, or combinations thereof.

Embodiment 36. The composition for use of any one of the preceding embodiments, wherein the anticonvulsant is chosen from lamotrigine, gabapentin, valproic acid, topiramate, famotodine, phenobarbital, diphenylhydantoin, phenytoin, mephenytoin, ethotoin, mephobarbital, primidone, carbamazepine, ethosuximide, methsuximide, phensuximide, trimethadione, benzodiazepine, phenacemide, acetazolamide, progabide, clonazepam, divalproex sodium, magnesium sulfate injection, metharbital, paramethadione, phenytoin sodium, valproate sodium, clobazam, sulthiame, dilantin, diphenylan and L-5-hydroxytrytophan, or a combination thereof.

Embodiment 37. The composition for use of any one of the preceding embodiments, wherein the subject is administered the pharmaceutical composition at a dose of from 40 mg/day to 100 mg/day of 4-aminopyridine, 4-aminopyridine derivative, or a combination thereof.

Embodiment 38. The composition for use of any one of the preceding embodiments, wherein the pharmaceutical composition comprises 4-aminopyridine, 3,4-diaminopyridine, 3-hydroxy-4-aminopyridine, or a combination thereof.

Embodiment 39. The composition for use of any one of the preceding embodiments, wherein the therapeutically effective amount is an amount sufficient to restore at least a portion of lost motor function or sensory function or cognitive function or visual function or auditory function, or a combination thereof in the subject, as compared to an untreated subject.

Embodiment 40. The composition for use of any one of the preceding embodiments, wherein the therapeutically effective amount is an amount that enhances repair and regeneration of stem cells or progenitor cells.

Embodiment 41. The composition for use of any one of the preceding embodiments, wherein the therapeutically effective amount is an amount that promotes neural cell generation, enhances cell survival, decreases Sox9 expression, chondroitin sulfate proteoglycan expression, reduces scarring, decreases lesion size, decreases oxidative damage, or combinations thereof.

Embodiment 42. The composition for use of any one of the preceding embodiments, wherein the therapeutically effective amount is an amount to decrease tissue damage due to a stroke, traumatic brain injury or other head injuries, injuries to the spinal cord (including central cord syndrome), and ischemic injury to the muscle, heart or other organs.

Embodiment 43. The composition for use of any one of the preceding embodiments, wherein the therapeutically effective amount is an amount to enhance recovery due to bone fracture.

Embodiment 44. The composition for use of any one of the preceding embodiments, wherein the therapeutically effective amount is an amount to prevent damage caused by burns.

Embodiment 45. The composition for use of any one of the preceding embodiments, wherein the therapeutically effective amount is an amount to decrease damage due to traumatic injury to the kidney, such as may occur due to ischemic injury or in automobile accidents.

Embodiment 46. The composition for use of any one of the preceding embodiments, wherein the therapeutically effective amount is an amount to treat multi-site and/or multi-organ traumatic injury.

Embodiment 47. A composition for use in treating an acute spinal cord injury in a subject, comprising: administering to the subject a therapeutically effective amount of a pharmaceutical composition comprising 4-aminopyridine, or a 4-aminopyridine derivative, or a combination thereof.

Embodiment 48. A composition for use in treating acute injury during surgery, comprising: administering to a subject undergoing surgery a therapeutically effective amount of a pharmaceutical composition comprising 4-aminopyridine, or a 4-aminopyridine derivative, or a combination thereof.

Embodiment 49. The composition for use of any one of the preceding embodiment, wherein the pharmaceutical composition is administered beginning at from 24 hours before surgery to 24 hours after surgery.

Embodiment 50. The composition for use of any one of the preceding embodiments, wherein the pharmaceutical composition is administered by periodic doses of the pharmaceutical composition.

Embodiment 51. The composition for use of any one of the preceding embodiments, where repeated administration is by continuous, sustained release of the pharmaceutical composition.

Embodiment 52. The composition for use of any one of the preceding embodiments, wherein the pharmaceutical composition is administered to the subject by intraperitoneal injection, intravenous injection, intramuscular injection, intrathecal injection, subcutaneous injection, sublingual administration, inhalation, oral administration, transdermal administration, or a combination thereof.

Embodiment 53. The composition for use of any one of the preceding embodiments, wherein the pharmaceutical composition is administered by implantation or insertion of a device into the subject.

Embodiment 54. The composition for use of any one of the preceding embodiments, wherein the pharmaceutical composition provides sustained release of the 4-aminopyridine, 4-aminopyridine derivative, or a combination thereof.

Embodiment 55. The composition for use of any one of the preceding embodiments, wherein the subject is administered the pharmaceutical composition at a dose of from 5 mg/day to 40 mg/day of 4-aminopyridine, 4-aminopyridine derivative, or a combination thereof.

Embodiment 56. The composition for use of any one of the preceding embodiments, wherein the pharmaceutical composition is administered in combination with an additional therapeutic agent.

Embodiment 57. The composition for use of any one of the preceding embodiments, wherein the pharmaceutical composition is administered in combination with an anticonvulsant.

Embodiment 58. The composition for use of any one of the preceding embodiments, wherein the pharmaceutical composition is administered at a dosage level of from about 30 mg to about 200 mg per day in combination with an anticonvulsant.

Embodiment 59. The composition for use of any one of the preceding embodiments, wherein the anticonvulsant is chosen from a barbiturate, a benzodiazepine, a bromide, a carbamate, a carboxamide, a fatty acid, a fructose derivative, a GABA analog, a hydantoin, an oxazolidinedione, a proprionate, a pyrimidinedione, a pyrrolidine, a succinimide, a sulfonamide, a triazine, a urea, or a valproylamide, or combinations thereof.

Embodiment 60. The composition for use of any one of the preceding embodiments, wherein the anticonvulsant is chosen from lamotrigine, gabapentin, valproic acid, topiramate, famotodine, phenobarbital, diphenylhydantoin, phenytoin, mephenytoin, ethotoin, mephobarbital, primidone, carbamazepine, ethosuximide, methsuximide, phensuximide, trimethadione, benzodiazepine, phenacemide, acetazolamide, progabide, clonazepam, divalproex sodium, magnesium sulfate injection, metharbital, paramethadione, phenytoin sodium, valproate sodium, clobazam, sulthiame, dilantin, diphenylan and L-5-hydroxytrytophan, or a combination thereof.

Embodiment 61. The composition for use of any one of the preceding embodiments, wherein the subject is administered the pharmaceutical composition at a dose of from 40 mg/day to 100 mg/day of 4-aminopyridine, 4-aminopyridine derivative, or a combination thereof.

Embodiment 62. The composition for use of any one of the preceding embodiments, wherein the pharmaceutical composition comprises 4-aminopyridine, 3,4-diaminopyridine, 3-hydroxy-4-aminopyridine, or a combination thereof.

Embodiment 63. The composition for use of any one of the preceding embodiments, wherein the therapeutically effective amount is an amount sufficient to restore at least a portion of lost motor function or sensory function or cognitive function or visual function or auditory function, or a combination thereof in the subject, as compared to an untreated subject.

Embodiment 64. The composition for use of any one of the preceding embodiments, wherein the therapeutically effective amount is an amount that enhances repair and regeneration of stem cells or progenitor cells.

Embodiment 65. The composition for use of any one of the preceding embodiments, wherein the therapeutically effective amount is an amount that promotes neural cell generation, enhances cell survival, decreases Sox9 expression, chondroitin sulfate proteoglycan expression, reduces scarring, decreases lesion size, decreases oxidative damage, or combinations thereof.

Embodiment 66. The composition for use of any one of the preceding embodiments, wherein the surgery is neurosurgery.

Embodiment 67. A composition for use in preventing muscle atrophy due to a traumatic injury in a subject, comprising: administering to the subject a therapeutically effective amount of a pharmaceutical composition comprising 4-aminopyridine, or a 4-aminopyridine derivative, or a combination thereof.

Embodiment 68. The composition for use of any one of the preceding embodiments, wherein the traumatic injury is to the peripheral nervous system, the central nervous system, or to the heart. It is intended that the specification and examples be considered as exemplary only, with a true scope and spirit of the invention being indicated by the following claims.

## Claims

1. A pharmaceutical composition comprising 4-aminopyridine, or a 4-aminopyridine derivative, or a combination thereof for use in treating an acute traumatic injury in a subject; comprising administering to the subject a therapeutically effective amount of said pharmaceutical composition within 2 weeks after the acute traumatic injury, further comprising repeatedly administering the pharmaceutical composition for between 1 to 16 weeks after the acute traumatic injury;
wherein 4-aminopyridine and the derivative thereof are represented by a structure according to Formula I: 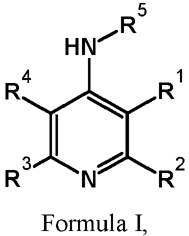 wherein R¹, R², R³, R⁴, and R⁵ are each independently selected from hydrogen, halogen, amine, hydroxyl, alkoxy, carboxyl, or C₁-C₆ alkyl.

2. The pharmaceutical composition for use as claimed in claim 1, wherein the acute traumatic injury is to an excitable tissue.

3. The pharmaceutical composition for use as claimed in claim 1, where the acute traumatic injury is to non-excitable tissue.

4. The pharmaceutical composition for use as claimed in any one of claims 1-3, wherein said pharmaceutical composition is administered to the subject by intraperitoneal injection, intravenous injection, intramuscular injection, intrathecal injection, subcutaneous injection, sublingual administration, inhalation, oral administration, transdermal administration, or a combination thereof.

5. The pharmaceutical composition for use as claimed in any one of claims 1-4, wherein the subject is administered said pharmaceutical composition at a dose of from 5 mg/day to 40 mg/day of 4-aminopyridine, 4-aminopyridine derivative, or a combination thereof.

6. The pharmaceutical composition for use as claimed in any one of claims 1-5, wherein said pharmaceutical composition is administered in combination with an additional therapeutic agent.

7. The pharmaceutical composition for use as claimed in any one of claims 1-6, wherein said pharmaceutical composition is administered in combination with an anticonvulsant.

8. The pharmaceutical composition for use as claimed in claim 7, wherein the anticonvulsant is chosen from a barbiturate, a benzodiazepine, a bromide, a carbamate, a carboxamide, a fatty acid, a fructose derivative, a GABA analog, a hydantoin, an oxazolidinedione, a proprionate, a pyrimidinedione, a pyrrolidine, a succinimide, a sulfonamide, a triazine, a urea, or a valproylamide, or combinations thereof.

9. The pharmaceutical composition for use as claimed in any one of claims 6-8, wherein the subject is administered said pharmaceutical composition at a dose of from 40 mg/day to 100 mg/day of 4-aminopyridine, 4-aminopyridine derivative, or a combination thereof.

10. The pharmaceutical composition for use as claimed in any one of claims 1-9, wherein the therapeutically effective amount is 5 mg or greater, such as 10 mg or greater.

11. The pharmaceutical composition for use as claimed in any one of claims 1-10, wherein the therapeutically effective amount is 50 mg or greater, such as 95 mg or greater.

12. The pharmaceutical composition for use as claimed in any one of claims 1-11, wherein the injury is a multi-site and/or multi-organ traumatic injury.

## Patentansprüche

1. Pharmazeutische Zusammensetzung, die 4-Aminopyridin oder ein 4-Aminopyridinderivat oder eine Kombination davon umfasst, zur Verwendung beim Behandeln einer akuten traumatischen Verletzung bei einem Subjekt; umfassend Verabreichen einer therapeutisch wirksamen Menge der pharmazeutischen Zusammensetzung innerhalb von 2 Wochen nach der akuten traumatischen Verletzung an das Subjekt, weiter umfassend wiederholtes Verabreichen der pharmazeutischen Zusammensetzung über 1 bis 16 Wochen nach der akuten traumatischen Verletzung;
wobei 4-Aminopyridin und sein Derivat durch eine Struktur gemäß der Formel I dargestellt sind: 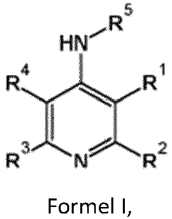 wobei R¹, R², R³, R⁴ und R⁵ jeweils unabhängig ausgewählt sind aus Wasserstoff, Halogen, Amin, Hydroxyl, Alkoxy, Carboxyl oder C₁-C₆-Alkyl.

2. Pharmazeutische Zusammensetzung zur Verwendung nach Anspruch 1, wobei die akute traumatische Verletzung an einem reizbaren Gewebe vorliegt.

3. Pharmazeutische Zusammensetzung zur Verwendung nach Anspruch 1, wo die akute traumatische Verletzung an einem nicht reizbaren Gewebe vorliegt.

4. Pharmazeutische Zusammensetzung zur Verwendung nach einem der Ansprüche 1-3, wobei die pharmazeutische Zusammensetzung dem Subjekt durch intraperitoneale Injektion, intravenöse Injektion, intramuskuläre Injektion, intrathekale Injektion, subkutane Injektion, sublinguale Verabreichung, Inhalation, orale Verabreichung, transdermale Verabreichung oder eine Kombination davon verabreicht wird.

5. Pharmazeutische Zusammensetzung zur Verwendung nach einem der Ansprüche 1-4, wobei dem Subjekt die pharmazeutische Zusammensetzung bei einer Dosis von 5 mg/Tag bis 40 mg/Tag von 4-Aminopyridin, 4-Aminopyridinderivat oder einer Kombination davon verabreicht wird.

6. Pharmazeutische Zusammensetzung zur Verwendung nach einem der Ansprüche 1-5, wobei die pharmazeutische Zusammensetzung in Kombination mit einem zusätzlichen therapeutischen Mittel verabreicht wird.

7. Pharmazeutische Zusammensetzung zur Verwendung nach einem der Ansprüche 1-6, wobei die pharmazeutische Zusammensetzung in Kombination mit einem krampflösenden Mittel verabreicht wird.

8. Pharmazeutische Zusammensetzung zur Verwendung nach Anspruch 7, wobei das krampflösende Mittel ausgewählt ist aus einem Barbiturat, einem Benzodiazepin, einem Bromid, einem Carbamat, einem Carboxamid, einer Fettsäure, einem Fructosederivat, einem GABA-Analog, einem Hydantoin, einem Oxazolidindion, einem Proprionat, einem Pyrimidindion, einem Pyrrolidin, einem Succinimid, einem Sulfonamid, einem Triazin, einem Harnstoff oder einem Valproylamid oder Kombinationen davon.

9. Pharmazeutische Zusammensetzung zur Verwendung nach einem der Ansprüche 6-8, wobei dem Subjekt die pharmazeutische Zusammensetzung bei einer Dosis von 40 mg/Tag bis 100 mg/Tag von 4-Aminopyridin, 4-Aminopyridinderivat oder einer Kombination davon verabreicht wird.

10. Pharmazeutische Zusammensetzung zur Verwendung nach einem der Ansprüche 1-9, wobei die therapeutisch wirksame Menge 5 mg oder größer, wie 10 mg oder größer ist.

11. Pharmazeutische Zusammensetzung zur Verwendung nach einem der Ansprüche 1-10, wobei die therapeutisch wirksame Menge 50 mg oder größer, wie 95 mg oder größer ist.

12. Pharmazeutische Zusammensetzung zur Verwendung nach einem der Ansprüche 1-11, wobei die Verletzung eine traumatische Verletzung an mehreren Stellen und/oder mehreren Organen ist.

## Revendications

1. Composition pharmaceutique comprenant de la 4-aminopyridine, ou un dérivé de 4-aminopyridine, ou une combinaison de ceux-ci pour utilisation dans le traitement d'une lésion traumatique aiguë chez un sujet ; comprenant l'administration au sujet d'une quantité thérapeutiquement efficace de ladite composition pharmaceutique dans les 2 semaines suivant la lésion traumatique aiguë, comprenant en outre l'administration répétée de la composition pharmaceutique pendant entre 1 et 16 semaines suivant la lésion traumatique aiguë ;
dans laquelle la 4-aminopyridine ou le dérivé de celle-ci sont représentés par une structure selon la formule I : 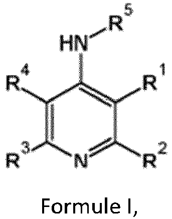 dans laquelle R¹, R², R³, R⁴, et R⁵ sont chacun sélectionné indépendamment parmi hydrogène, halogène, amine, hydroxyle, alcoxy, carboxyle ou alkyle en C₁-C₆.

2. Composition pharmaceutique pour utilisation selon la revendication 1, dans laquelle la lésion traumatique aiguë touche un tissu excitable.

3. Composition pharmaceutique pour utilisation selon la revendication 1, dans laquelle la lésion traumatique aiguë touche un tissu non excitable.

4. Composition pharmaceutique pour utilisation selon l'une quelconque des revendications 1-3, dans laquelle ladite composition pharmaceutique est administrée au sujet par injection intrapéritonéale, injection intraveineuse, injection intramusculaire, injection intrathécale, injection sous-cutanée, administration sublinguale, inhalation, administration orale, administration transdermique ou une combinaison de celles-ci.

5. Composition pharmaceutique pour utilisation selon l'une quelconque des revendications 1-4, dans laquelle il est administré au sujet ladite composition pharmaceutique à raison de 5 mg/jour à 40 mg/jour de 4-aminopyridine, dérivé de 4-aminopyridine ou une combinaison de ceux-ci.

6. Composition pharmaceutique pour utilisation selon l'une quelconque des revendications 1-5, dans laquelle ladite composition pharmaceutique est administrée en combinaison avec un agent thérapeutique supplémentaire.

7. Composition pharmaceutique pour utilisation selon l'une quelconque des revendications 1-6, dans laquelle ladite composition pharmaceutique est administrée en combinaison avec un anticonvulsivant.

8. Composition pharmaceutique pour utilisation selon la revendication 7, dans laquelle l'anticonvulsivant est choisi parmi un barbiturique, une benzodiazépine, un bromure, un carbamate, un carboxamide, un acide gras, un dérivé de fructose, un analogue de GABA, une hydantoïne, une oxazolidinedione, un proprionate, une pyrimidinedione, une pyrrolidine, un succinimide, un sulfonamide, une triazine, une urée, ou un valproylamide, ou des combinaisons de ceux-ci.

9. Composition pharmaceutique pour utilisation selon l'une quelconque des revendications 6-8, dans laquelle il est administré au sujet ladite composition pharmaceutique à raison de 40 mg/jour à 100 mg/jour de 4-aminopyridine, dérivé de 4-aminopyridine ou une combinaison de ceux-ci.

10. Composition pharmaceutique pour utilisation selon l'une quelconque des revendications 1-9, dans laquelle la quantité thérapeutiquement efficace est supérieure ou égale à 5 mg, par exemple supérieure ou égale à 10 mg.

11. Composition pharmaceutique pour utilisation selon l'une quelconque des revendications 1-10, dans laquelle la quantité thérapeutiquement efficace est supérieure ou égale à 50 mg, par exemple supérieure ou égale à 95 mg.

12. Composition pharmaceutique pour utilisation selon l'une quelconque des revendications 1-11, dans laquelle la lésion est une lésion traumatique touchant plusieurs sites et/ou plusieurs organes.
